# FASCICULE DE BREVET EUROPEEN

(11) **EP 4 007 499 B1**
(45) Date de publication et mention de la délivrance du brevet: **20.12.2023**
(21) Numéro de dépôt: 20746238.3
(22) Date de dépôt: 31.07.2020
(51) Int. Cl.: A23K 20/147, A23J 3/34, A23K 50/40, A23L 33/18, A61K 38/01, A23L 33/115, A23L 33/12, A61K 31/202, A23J 3/04

(54) **HYDROLYSAT PROTEIQUE ISSU DE POISSONS BLEUS**
PROTEINHYDROLYSAT AUS BLAUFISCHEN
PROTEIN HYDROLYSATE OBTAINED FROM BLUE FISH

(30) Priorité: 01.08.2019 FR 1908837
(43) Date de publication de la demande: 08.06.2022
(73) Titulaire: Spécialités Pet Food, 56250 Elven (FR); UNIVERSITE DE BORDEAUX, 33000 Bordeaux (FR); Université de Bretagne Occidentale, 29200 Brest (FR); CENTRE NATIONAL DE LA RECHERCHE SCIENTIFIQUE (CNRS), 75016 Paris (FR); Institut de Recherche pour le Développement (IRD), 13002 Marseille 2 (FR); Institut Polytechnique de Bordeaux, 33400 Talence (FR); Abyss Ingredients, 56850 Caudan (FR); Institut Français de Recherche pour l'Exploitation de la Mer (IFREMER), 29280 Plouzane (FR); Institut national de recherche pour l'agriculture, l'alimentation et l'environnement, 75007 Paris (FR)
(72) Inventeur: GUERARD, Fabienne, 29900 CONCARNEAU (FR); OROY, Cloé, 27950 SAINT-MARCEL (FR); LEPOUDERE, Anne, 56380 GUER (FR); CHATAIGNER, Mathilde, 76230 QUIMCAMPOIX (FR); ALLAUME, Patrick, 56850 CAUDAN (FR); DINEL, Anne-Laure, 33700 MERIGNAC (FR); JOFFRE, Corinne, 33600 PESSAC (FR); PALLET, Véronique, 33600 PESSAC (FR); LE GRAND, Fabienne, 29820 GUILERS (FR)
(74) Mandataire: Oak & Fox
(86) Numéro de dépôt international: PCT/EP2020/071717
(87) Numéro de publication internationale: WO 2021/019093

(56) Documents cités:
- EP-A1- 2 316 426
- FR-A1- 2 835 703
- FR-A1- 2 927 336
- FR-A1- 2 966 016
- FR-A1- 3 036 923
- FR-B1- 2 835 703
- US-A1- 2008 070 870
- US-A1- 2019 037 882

## Description

### DOMAINE DE L'INVENTION

La présente invention concerne le domaine des hydrolysats protéiques fonctionnels. Plus précisément, elle concerne un hydrolysat protéique obtenu à partir d'au moins une source protéique issue de poissons bleus comprenant (i) un degré d'hydrolyse (DH) d'au moins 10%, (ii) au moins 80% de protéines solubles dans l'eau dont le poids moléculaire est inférieur à 1000 Da, (iii) au moins 0.3% de phospholipides, et (iv) au moins 0.5% de DHA et EPA. Elle concerne encore des compositions alimentaires ou pharmaceutiques comprenant ledit hydrolysat protéique et leur utilisation pour la prévention et/ou le traitement des troubles cognitifs légers liés à l'âge.

### ETAT DE LA TECHNIQUE

Le vieillissement démographique constitue l'un des plus grands défis économiques et sociaux du XXIe siècle (1). D'ici à 2040, le nombre de personnes âgées de plus de 65 ans dans le monde devrait plus que doubler, passant de 506 millions en 2008 à 1,3 milliard de personnes, selon une étude américaine de 2009 (2). D'ici 2025 en Europe, plus de 148 millions d'habitants auront plus de 65 ans (>20 % de la population) contre 120 millions en 2010, avec une augmentation particulièrement rapide du nombre d'octogénaires. Le vieillissement s'accompagne chez l'Homme de l'apparition de symptômes spécifiques, notamment d'un déclin cognitif lié au vieillissement cérébral (3). Il s'agit d'une altération non pathologique mais significative des fonctions cérébrales incluant notamment des troubles mnésiques et de la vigilance pouvant conduire à une perte d'autonomie et pouvant parfois annoncer des pathologies neurodégénératives. Trouver des solutions permettant de vieillir en bonne santé le plus longtemps possible est donc primordial et constitue pour les pays développés un véritable enjeu économique, sociétal et de santé publique. Le concept de « healthy aging » (4) ou « vieillissement réussi » intègre ces différents aspects et l'ère de la médecine des 4P (plus préventive, prédictive, personnalisée, participative) dans laquelle nous rentrons devrait faire une part belle dans les années à venir à la prévention, notamment par la nutrition, et à la prise en charge par chacun de sa santé.

De manière similaire, l'espérance de vie augmente chez les animaux de compagnie, notamment celle des chats et des chiens, et ce, de manière continue, grâce à l'amélioration de la nutrition et des soins apportés. Aux Etats-Unis par exemple, la longévité des chiens atteignait en moyenne 11 ans en 2012 (+ 0.5 an par rapport à 2002), elle était de 12 ans cette même année pour les chats (+ 1 an par rapport à 2002) (5). Aux Etats-Unis toujours, on estime à 30-40 % de la population totale canine, la proportion de chiens de plus de 7 ans. En Europe, l'estimation moins précise porte cette proportion à 25-45 %.

Comme chez l'Homme, l'augmentation de l'espérance de vie des animaux de compagnie soulève de vrais défis quant au maintien de conditions de vie satisfaisantes pour les individus âgés. L'un des principaux enjeux est notamment de réduire la fréquence des maladies neurodégénératives liées à l'âge, ou du moins de les retarder. Parmi ces maladies, le syndrome de dysfonctionnement cognitif (SDC) est bien caractérisé chez le chien. Ses manifestations comportementales sont la perte d'orientation, les altérations de la relation avec le maître et les changements des cycles sommeil-éveil (6). Ces effets sont également constatés chez les chats âgés (6). Des études épidémiologiques réalisées indiquent que le SDC toucherait 5% des chiens âgés de 10 à 12 ans, 23% des chiens âgés de 12 à 14 ans et enfin, 41% des chiens âgés de plus de 14 ans (7).

Pour faire face à ces enjeux, il existe un besoin pour de nouveaux produits capables de prévenir, chez l'Homme comme chez l'animal, le déclin cognitif lié à l'âge ainsi que les troubles qui y sont associés, et ainsi permettre de maintenir plus longtemps la qualité de vie mais aussi l'autonomie des individus âgés.

Les effets bénéfiques de la consommation de poissons gras et maigres sont connus de longue date, et aujourd'hui il existe un réel intérêt des consommateurs pour les produits de la mer. Il est connu que les poissons gras en général, et les poissons bleus en particulier, sont riches en acides gras polyinsaturés (AGPI), notamment EPA (acide eicosapentaénoïque) et DHA (acide docosahexaénoïque), en phospholipides, ainsi qu'en protéines.

Toutefois, à la connaissance des inventeurs, il n'existe pas sur le marché de la nutraceutique ou des aliments pour animaux de compagnie, de produit ou ingrédient renfermant de manière non négligeable ces actifs d'intérêt pour le cerveau, obtenu par un procédé éco-respectueux en partant d'une ressource marine unique, en particulier de poissons bleus comme la sardine.

Les inventeurs ont ainsi développé un procédé d'hydrolyse particulièrement innovant, permettant d'obtenir des hydrolysats de poissons bleus présentant des effets bénéfiques pour la prévention du déclin cognitif lié à l'âge. Riche en peptides bioactifs associés à du DHA et des phospholipides, un tel hydrolysat présente notamment des effets avantageux au niveau de la neuroprotection, de l'amélioration des capacités cognitives et de réduction du stress/de l'anxiété.

Des hydrolysats de protéines de poissons, en particulier de protéines de maquereau, sont décrits dans FR 2 966 016, par exemple.

### EXPOSE DE L'INVENTION

Un but de l'invention est donc de fournir un produit « 3 en 1 » renfermant au moins 3 actifs d'intérêt pour le cerveau, notamment des peptides, des phospholipides et du DHA, permettant de prévenir et/ou traiter, chez l'Homme comme chez l'animal, le déclin cognitif lié à l'âge et les troubles qui y sont associés.

Il est à cet effet proposé, selon un premier aspect de l'invention, un hydrolysat protéique obtenu à partir d'au moins une source protéique issue de poissons bleus, notamment au moins issue de têtes de poissons bleus, comprenant :
(i) un degré d'hydrolyse (DH) d'au moins 10%,
(ii) au moins 80% de protéines solubles dans l'eau dont le poids moléculaire est inférieur à 1000 Da,
(iii) au moins 0.3% de phospholipides, et
(iv) au moins 0.5% de DHA et EPA.

L'invention porte encore sur une composition alimentaire comprenant au moins un hydrolysat protéique selon l'invention, caractérisée en ce qu'il s'agit d'un aliment complet ou d'un complément alimentaire, notamment un aliment fonctionnel ou nutraceutique, ladite composition alimentaire pouvant être à destination de l'Homme ou de l'animal.

Un autre objet de l'invention concerne un procédé de préparation d'un hydrolysat protéique selon l'invention comprenant les étapes de :
a. fourniture d'une source protéique ;
b. broyage de ladite source protéique ;
c. optionnellement, ajustement du pH ;
d. ajout d'au moins une enzyme d'hydrolyse ;
e. chauffage ;
f. séparation ;
g. séchage.

L'invention porte finalement sur un hydrolysat protéique selon l'invention ou une composition pharmaceutique en contenant, pour une utilisation comme médicament.

### DESCRIPTION DES FIGURES

Toutes les figures illustrent des modes de réalisation d'hydrolysats purs en poudre conformément à la présente invention.
**Figure 1a** **:** Effet in vitro de l'hydrolysat H1 ou du DHA sur l'expression de la cytokine pro-inflammatoire IL-6 dans les cellules microgliales BV2 après 2h, 6h et 24h de traitement au LPS (n=9 ; **p<0.01, ***p<0.001 Contrôle LPS vs Traitement LPS, $p<0.05, $$p<0.01 DHA LPS vs H1 LPS).
**Figure 1b** : Effet in vitro de l'hydrolysat H1 ou du DHA sur l'expression de la cytokine pro-inflammatoire IL-113 dans les cellules microgliales BV2 après 2h, 6h et 24h de traitement au LPS (n=9 ; **p<0.01, ***p<0.001 Contrôle LPS vs Traitement LPS, $p<0.05, $$p<0.01 DHA LPS vs H1 LPS).
**Figure 1c** **:** Effet in vitro de l'hydrolysat H1 ou du DHA sur l'expression de la cytokine pro-inflammatoire TNF-α dans les cellules microgliales BV2 après 2h, 6h et 24h de traitement au LPS (n=9 ; **p<0.01, ***p<0.001 Contrôle LPS vs Traitement LPS, $p<0.05, $$p<0.01 DHA LPS vs H1 LPS).
**Figure 2** : Effet in vitro de l'hydrolysat H2 sur l'expression des cytokines pro-inflammatoires IL-6, IL-1β et TNF-α et du facteur neurotrophique BDNF dans des cellules microgliales BV2 en co-culture avec des cellules neuronales HT22 traitées pendant 6h au LPS (n=11 ; *p<0.05, **p<0.01, ***p<0.001).
**Figure 3** : Effet in vitro de l'hydrolysat H2 sur l'expression des facteurs neurotrophiques BDNF et NGF dans des cellules neuronales HT22 en co-culture avec des cellules microgliales BV2 traitées pendant 6h au LPS (n=11).
**Figure 4** **:** Effet d'une supplémentation en hydrolysat H2 sur le comportement de type anxieux et le taux de corticostérone chez les souris jeunes et âgées (n=11-13 par groupe ; $p<0.05, $$p<0.01, ; effet régime **p<0.01).
**Figure 5** : Effet d'une supplémentation en hydrolysat H2, avec ou sans ajout de DHA, sur la réactivité au stress chez les souris jeunes et âgées (n=11-13 par groupe ; $p<0.05, $$p<0.01, $$$p<0.001 Jeune vs Agé ; *p<0.05, **p<0.01, ***p<0.001 Traitement vs Traitement).
**Figure 6** : Effet d'une supplémentation en hydrolysat H2 sur l'expression des gènes de réponse au stress dans l'hypothalamus des souris jeunes et âgées supplémentées pendant 11 semaines (n=7-9 par groupe ; *p<0.05, **p<0.01, #p=0.0624).
**Figure 7** : Effet d'une supplémentation en hydrolysat H2, avec ou sans ajout de DHA, sur la mémoire hippocampique à court terme évaluée par la mesure de l'indice de reconnaissance du bras nouveau dans le test du labyrinthe en Y (n=11-13 par groupe ; *p<0.05, **p<0.01, ***p<0.001, #p=0.0596 vs Hasard (33%)).
**Figure 8** : Effet d'une supplémentation en hydrolysat H2 sur l'apprentissage spatial et la mémoire hippocampique à long-terme. (A) Distance parcourue pour atteindre la plateforme au cours des 4 jours d'apprentissage spatial ($p<0.05). (B) Pourcentage de distance parcourue dans les quadrants durant le test standard « probe test » ($$p<0.01 ; $$$p<0.001 vs Hasard (25%) ; *p<0.05, **p<0.01 ; ***p<0.001 vs QO (cible)), (n=11-13 par groupe).
**Figure 9** : Effet d'une supplémentation en hydrolysat H2 sur le pourcentage de stratégies spatiales utilisées lors de l'apprentissage spatial chez les souris jeunes et âgées (n=11-13 par groupe ; effet régime *p<0.05, effet âge $p<0.01).
**Figure 10** : Effet d'une supplémentation en hydrolysat H2 sur l'expression des marqueurs microgliaux dans les hippocampes des souris supplémentées pendant 11 semaines, (n=7-9 par groupe pour CD11b et n=4 par groupe pour Iba1 ; effet régime *p<0.05, effet âge $$p<0.01).
**Figure 11** : Effet d'une supplémentation en hydrolysat H2 sur l'expression génique des enzymes de synthèse impliquées dans la béta-oxydation mitochondriale et péroxysomale dans l'hippocampe de souris supplémentées pendant 11 semaines (n=7-8 par groupe ; *p<0.05, **p<0.01, ***p<0.001).
**Figure 12** : Effet d'une supplémentation en hydrolysat H2 sur l'expression génique des enzymes de synthèse impliquées dans les défenses anti-oxydantes dans l'hippocampe de souris supplémentées pendant 11 semaines (n=7-8 par groupe ; effet régime *p<0.05 ; effet âge $p<0.05).
**Figure 13** : Effet d'une supplémentation de 18 jours en hydrolysat H2 ou en DHA sur l'expression génique des cytokines pro-inflammatoires dans l'hippocampe de souris en réponse au LPS (n=4-6 par groupe ; ***p<0.001).
**Figure 14** : Effet d'une supplémentation de 18 jours en hydrolysat H2 ou en DHA sur l'expression génique de COX-2 dans l'hippocampe de souris en réponse au LPS (n=4-6 par groupe).
**Figure 15** **:** Effet d'une supplémentation de 18 jours en hydrolysat H2 ou en DHA sur l'expression protéique d'IkB dans l'hippocampe de souris en réponse au LPS (n=5-6 par groupe ; *p<0.05, **p<0.01).
**Figure 16** : Effet d'une supplémentation de 18 jours en hydrolysat H2 ou en DHA sur la teneur en oxylipines dans l'hippocampe de souris en réponse au LPS, (n=4-6 par groupe ; les valeurs avec des exposants (a, b, c, d, e) diffèrent significativement).
**Figure 17** **:** Effet d'une supplémentation de 18 jours en hydrolysat H2 ou en DHA sur l'expression génique des neurotrophines BDNF et NGF dans l'hippocampe de souris en réponse au LPS (n=4-6 par groupe ; *p<0.05, **p<0.01, ***p<0.001).

### DEFINITIONS

Sauf indication contraire spécifique, les pourcentages sont exprimés ici en poids d'un produit de référence.

Dans la présente description, les intervalles sont définis de façon abrégée afin d'éviter de les reproduire intégralement et de décrire chacune des valeurs et toutes les valeurs de cet intervalle. Toute valeur appropriée dans l'intervalle peut être choisie comme la valeur supérieure, la valeur inférieure ou les valeurs terminales de l'intervalle. Par exemple, un intervalle de 0,1 à 1,0 représente les valeurs terminales de 0,1 et 1,0, ainsi que les valeurs intermédiaires de 0,2, 0,3, 0,4, 0,5, 0,6, 0,7, 0,8, 0,9, et tous les intervalles intermédiaires compris dans 0,1 à 1,0, tels que 0,2 à 0,5, 0,2 à 0,8, 0,7 à 1,0, etc. Un intervalle défini comme étant « compris entre la valeur A et la valeur B » inclut les valeurs A et B et est donc équivalent à un intervalle « allant de la valeur A à la valeur B ». En outre, l'expression « au moins » comprend la valeur énoncée ci-après. Par exemple, « au moins 5 % » doit être compris comme comprenant également « 5 % ». L'expression « un maximum de » comprend la valeur énoncée ci-après. Par exemple, « un maximum de 5 % » doit être compris comme comprenant également « 5 % ».

En outre, dans la présente invention, les valeurs mesurables telles qu'une quantité, doivent être comprises comme comprenant les écarts types qui peuvent être facilement déterminés par l'homme du métier dans le domaine technique de référence. De préférence, ces valeurs sont destinées à comprendre des variations de ± 5 %.

Telle qu'elle est utilisée dans tout ce document, la forme au singulier d'un mot comprend le pluriel et inversement, sauf si le contexte indique clairement le contraire. Ainsi, les références « un », « une » et « le/la » comprennent généralement les pluriels des termes respectifs. Par exemple, une référence à un « procédé » ou un « aliment » comprend une pluralité de ces « procédés » ou « aliments ». De façon similaire, les mots « comprendre », « comprend » et « comprenant » seront interprétés de manière inclusive. De même, les termes « inclure », « incluant » et « ou » doivent tous être considérés comme inclusifs. Tous ces termes devront toutefois être considérés comme englobant des modes de réalisation exclusifs qui peuvent également être désignés en utilisant des termes tels que « consiste en ».

Les procédés et compositions et autres modes de réalisation illustrés ici ne sont pas limités à des méthodologies, protocoles et réactifs particuliers qui sont décrits ici car ils peuvent varier comme le comprendra l'homme du métier.

Sauf indication contraire, tous les termes techniques et scientifiques, les termes utilisés dans l'art et les acronymes utilisés ici ont les significations communément admises par l'homme du métier dans le/les domaine(s) de l'invention, ou dans le/les domaine(s) dans lesquels le terme est utilisé. Bien que toute composition, tout procédé, article fabriqué ou autres moyens ou matériaux similaires ou équivalents à ceux décrits ici puisse être utilisé(e) dans la mise en pratique de la présente invention, les compositions, procédés, articles fabriqués préférée(s) ou autres moyens ou matériaux sont décrits ici.

### DESCRIPTION DETAILLEE DE L'INVENTION

Les inventeurs ont mis au point un hydrolysat de poissons bleus. Les composés d'intérêt des poissons bleus, et notamment les protéines, le DHA et les phospholipides, sont combinés dans l'hydrolysat selon l'invention. De manière particulièrement avantageuse, les inventeurs ont optimisé les proportions de ces composés d'intérêt afin de permettre l'obtention d'effets biologiques bénéfiques. Plus particulièrement, l'hydrolysat comprend une large proportion protéique, principalement sous forme de peptides. Cet hydrolysat, riche donc en peptides bioactifs et comprenant des phospholipides et du DHA, présente notamment des effets bénéfiques dans la prévention du déclin cognitif et de l'anxiété, en particulier lié à l'âge.

### Hydrolysat protéique

Dans le cadre de la présente invention, plusieurs méthodes de calcul ont été employées, notamment pour déterminer les profils de poids moléculaire des protéines et/ou peptides, les quantités de protéines, notamment protéines solubles, les quantités de phospholipides, DHA, EPA et autres composants dudit hydrolysat. Les méthodes de calcul et/ou articles de références détaillant lesdites méthodes sont toutes présentées à l'Exemple 1.7.

Les valeurs des teneurs mentionnées dans le présent texte s'entendent par référence à l'hydrolysat poudre séché sans l'aide d'un support de séchage (on parlera d' « hydrolysat pur en poudre »). Or, dans le procédé d'obtention de l'hydrolysat, un support de séchage pourra avantageusement être utilisé pour faciliter la mise en oeuvre de l'étape de séchage à grande échelle. En ce cas, on obtiendra un « hydrolysat poudre avec support » comprenant une proportion de support de séchage. Typiquement, on utilisera un support de séchage à hauteur de 2 à 20% par rapport au poids de l'hydrolysat liquide avant séchage. Ainsi, pour connaître les valeurs des teneurs de l'hydrolysat poudre avec support, il suffira d'adapter les valeurs des teneurs fournies dans le présent texte en référence à l'hydrolysat pur en poudre proportionnellement à la quantité d'hydrolysat pur en poudre contenue dans l'hydrolysat poudre avec support.

Selon un premier aspect, l'invention porte sur un hydrolysat protéique obtenu à partir d'au moins une source protéique issue de poissons bleus comprenant :
(i) un degré d'hydrolyse (DH) d'au moins 10%,
(ii) au moins 80% de protéines solubles dans l'eau dont le poids moléculaire est inférieur à 1000 Da,
(iii) au moins 0.3% de phospholipides, et
(iv) au moins 0.5% de DHA et EPA.

On entend par « **poisson bleu** » les poissons pélagiques de haute mer. Le poisson bleu tient son nom à la couleur de sa peau qui est influencée par l'accumulation de graisse, notamment les acides gras oméga 3, dans ses muscles. Les poissons bleus visés par la présente invention sont les poissons bleus de petite taille choisis parmi les poissons de la Famille *Clupeidae,* et notamment la sardine (*Sardina pilchardus, Sardinops* sp, *Sardinella sp.*) ou le hareng (*Clupea harengus*) ; l'anchois (*Engraulis encrasicolus*)*,* le maquereau (*Scomber scombrus*) et les poissons du genre *Trachurus.*

L'hydrolysat protéique de l'invention est ainsi obtenu par l'hydrolyse d'une source protéique issue de poissons bleus. On entend par « **degré d'hydrolyse** » (DH), le pourcentage de liens peptidiques coupés lors d'une hydrolyse protéique.

L'hydrolysat de l'invention présente un degré d'hydrolyse d'au moins 10%, ledit DH étant déterminé par la méthode pH-stat comme décrit par J. Adler-Nissen (17). De préférence, le degré d'hydrolyse est supérieur à 11%. En particulier, le degré d'hydrolyse de ladite source protéique est inférieur à 20%, de préférence inférieur à 18%, de préférence inférieure à 17%, de préférence encore inférieure à 16%, de préférence encore inférieur à 15%. L'obtention d'un tel degré d'hydrolyse permet avantageusement d'avoir les peptides bioactifs et les lipides d'intérêt dans l'hydrolysat selon l'invention.

L'hydrolysat selon l'invention comprend en particulier au moins 45%, de préférence au moins 50%, de préférence encore au moins 55%, de manière encore préférée au moins 60%, de manière préférée entre toutes au moins 65% de protéines totales (% en poids d'hydrolysat pur en poudre). Ledit hydrolysat comprend en particulier un maximum de 95%, de préférence un maximum de 90%, de préférence encore un maximum de 85%, de manière encore préférée un maximum 80% de protéines totales (% en poids d'hydrolysat pur en poudre).

Selon un mode de réalisation particulier, l'hydrolysat selon l'invention comprend au moins 45%, de préférence au moins 50%, de préférence encore au moins 55%, de manière encore préférée au moins 60%, de manière préférée entre toutes au moins 65% de protéines solubles (% en poids d'hydrolysat pur en poudre). Ledit hydrolysat comprend en particulier un maximum de 95%, de préférence un maximum de 90%, de préférence encore un maximum de 85%, de manière encore préférée un maximum 80%, de manière préférée entre toutes un maximum de 75% de protéines solubles (% en poids d'hydrolysat pur en poudre).

Un large spectre d'activités biologiques est attribué aux peptides composant les hydrolysats : activités anti-hypertensives, hypocholestérolémiantes, immuno-modulantes, anti-microbiennes, anti-oxydantes et opioïdes (ou anti-stress), etc. De façon générale, les peptides bioactifs comportent généralement de 4 à 20 acides aminés. Ils sont ainsi plus résistants à l'action des enzymes digestives tandis que leur taille contenue leur permet de franchir la barrière intestinale et d'être véhiculés par le sang. De manière avantageuse, l'hydrolysat protéique de l'invention est riche en peptides. Il comprend notamment au moins 80% de protéines solubles dans l'eau de poids moléculaire inférieur à 1000 daltons (Da) (peptides). Dans le cadre de la présente invention, l'expression « protéines de poids moléculaire de XX Da » désigne des acides aminés et/ou peptides et/ou protéines, selon la valeur de poids moléculaire. Les « peptides » sont notamment définis par un poids moléculaire inférieur à 1000 Da. En particulier, l'hydrolysat protéique de l'invention comprend au moins 80% de protéines solubles dans l'eau de poids moléculaire inférieur à 1000 Da, de préférence au moins 83% de protéines solubles dans l'eau de poids moléculaire inférieur à 1000 Da, de préférence encore au moins 84% de protéines solubles dans l'eau de poids moléculaire inférieur à 1000 Da, de manière encore préférée au moins 85% de protéines solubles dans l'eau de poids moléculaire inférieur à 1000 Da, de manière préférée entre toutes au moins 86% de protéines solubles dans l'eau de poids moléculaire inférieur à 1000 Da. Ledit hydrolysat contient un maximum de 95% de protéines solubles dans l'eau de poids moléculaire inférieur à 1000 Da, de préférence un maximum de 93% de protéines solubles dans l'eau de poids moléculaire inférieur à 1000 Da, de préférence encore un maximum de 92% de protéines solubles dans l'eau de poids moléculaire inférieur à 1000 Da, de manière encore préférée un maximum 91% de protéines solubles dans l'eau de poids moléculaire inférieur à 1000 Da, de manière préférée entre toutes un maximum de 90% de protéines solubles dans l'eau de poids moléculaire inférieur à 1000 Da.

Selon un mode de réalisation particulier, l'hydrolysat selon l'invention présente le profil de poids moléculaire suivant :
- au moins 50%, de préférence au moins 55%, de préférence encore au moins 60%, de préférence encore au moins 65%, de préférence encore au moins 68% de protéines solubles dans l'eau ayant un poids moléculaire inférieur à 500 Da, et de préférence un maximum de 95%, de préférence encore un maximum de 90%, de préférence encore un maximum de 85%, de préférence encore un maximum de 82%, de préférence encore un maximum de 80% de protéines solubles dans l'eau ayant un poids moléculaire inférieur à 500 Da ;
- au moins 8%, de préférence au moins 8.5%, de préférence encore au moins 9%, de préférence encore au moins 9.5%, de préférence encore au moins 10% de protéines solubles dans l'eau ayant un poids moléculaire compris entre 500 Da et 1000 Da, et de préférence un maximum de 35%, de préférence encore un maximum de 30%, de préférence encore un maximum de 25%, de préférence encore un maximum de 22%, de préférence encore un maximum de 20%, de préférence encore un maximum de 18%, de préférence encore un maximum de 17% de protéines solubles dans l'eau ayant un poids moléculaire compris entre 500 Da et 1000 Da;
- au moins 7%, de préférence au moins 7.5%, de préférence encore au moins 8%, de préférence encore au moins 8.5%, de préférence encore au moins 9% de protéines solubles dans l'eau ayant un poids moléculaire compris entre 1000 Da et 5000 Da, et de préférence un maximum de 20%, de préférence encore un maximum de 18%, de préférence encore un maximum de 16%, de préférence encore un maximum de 15%, de préférence encore un maximum de 14% de protéines solubles dans l'eau ayant un poids moléculaire compris entre 1000 Da et 5000 Da;
- au moins 0.10%, de préférence au moins 0.15%, de préférence encore au moins 0.20%, de préférence encore au moins 0.25%, de préférence encore au moins 0.30% de protéines solubles dans l'eau ayant un poids moléculaire supérieur à 5000 Da, et de préférence un maximum de 2.0%, de préférence encore un maximum de 1.5%, de préférence encore un maximum de 1.0%, de préférence encore un maximum de 0.8%, de préférence encore un maximum de 0.7% de protéines solubles dans l'eau ayant un poids moléculaire supérieur à 5000 Da.

L'hydrolysat comprend de préférence entre 12% et 35%, de préférence encore entre 15% et 32%, de préférence encore entre 17% et 30%, de manière encore préférée entre 20% et 28%, de manière préférée entre toutes entre 22% et 26% d'acides aminés libres par rapport aux protéines totales.

En particulier, l'hydrolysat comprend de préférence entre 0.3% et 2.0%, de préférence encore entre 0.4 et 1.8%, de préférence encore entre 0.5% et 1.5%, de manière encore préférée entre 0.6% et 1.3%, de manière préférée entre toutes entre 0.7% et 1.0% de tryptophane (% en poids d'hydrolysat pur en poudre).

En particulier, l'hydrolysat comprend de préférence entre 3.0% et 9.0%, de préférence encore entre 3.5% et 8.5%, de préférence encore entre 4.0% et 8.0%, de manière encore préférée entre 4.5% et 7.5%, de manière préférée entre toutes entre 5.0% et 7.0% de lysine (% en poids d'hydrolysat pur en poudre).

En particulier, l'hydrolysat comprend entre 3% et 20%, de préférence encore entre 5% et 18%, de préférence encore entre 7% et 16%, de manière encore préférée entre 9% et 15%, de manière préférée entre toutes entre 10% et 14% d'acides aminés branchés (% en poids d'hydrolysat pur en poudre). On entend par « acides aminés branchés » l'isoleucine, la leucine et la valine.

En particulier, l'hydrolysat comprend entre 0.5% et 8%, de préférence encore entre 1% et 7%, de préférence encore entre 1.5% et 6%, de manière encore préférée entre 1.8% et 5%, de manière préférée entre toutes entre 2% et 4% d'acides aminés soufrés (% en poids d'hydrolysat pur en poudre). On entend par « acides aminés soufrés » la cystine, la cystéine et la méthionine.

En particulier, l'hydrolysat comprend entre 15% et 45%, de préférence encore entre 17% et 42%, de préférence encore entre 20% et 39%, de manière encore préférée entre 23% et 36%, de manière préférée entre toutes entre 25% et 34% d'acides aminés essentiels (% en poids d'hydrolysat pur en poudre). En particulier, l'hydrolysat comprend entre 5% et 20%, de préférence encore entre 6% et 18%, de préférence encore entre 7% et 16%, de manière encore préférée entre 8% et 14%, de manière préférée entre toutes entre 9% et 12% d'acides aminés libres essentiels (% en poids d'hydrolysat pur en poudre). On entend par « acides aminés essentiels » la lysine, la méthionine, la cystine, la thréonine, le tryptophane, la phénylalanine, la tyrosine, la valine, la leucine, l'isoleucine (pour les Hommes).

L'hydrolysat selon l'invention présente notamment une teneur en matière grasse totale d'au moins 3%, de préférence d'au moins 4%, de préférence encore d'au moins 5%, de manière encore préférée au moins 6%, de manière préférée entre toutes au moins 7% (% en poids d'hydrolysat pur en poudre). Ledit hydrolysat comprend notamment une teneur en matière grasse totale inférieure ou égale à 20%, de préférence inférieure ou égale à 19%, de préférence encore inférieure ou égale à 18%, de manière encore préférée inférieure ou égale à 17%, de manière préférée entre toutes inférieure ou égale à 16% (% en poids d'hydrolysat pur en poudre).

Par « matière grasse totale » on entend le total des lipides neutres et des lipides polaires (ou phospholipides) ce qui correspond aux « lipides totaux ».

Parmi les lipides présents, l'hydrolysat protéique de l'invention contient avantageusement des phospholipides (ou lipides polaires). Les phospholipides sont des lipides structurels, constituants principaux des membranes. Les acides gras associés (C20 et C22) aux phospholipides ont un rôle essentiel dans la membrane plasmique puisqu'ils assurent l'amélioration de sa fluidité grâce à deux éléments, la longueur des chaînes et les rotations autorisées par les doubles liaisons.

L'hydrolysat protéique de l'invention comprend au moins 0.3% de phospholipides (% en poids d'hydrolysat pur en poudre). En particulier, il comprend au moins 0.35% de phospholipides, de préférence au moins 0.4% de phospholipides, de préférence encore au moins 0.45% de phospholipides, de manière encore préférée au moins 0.5% de phospholipides, de manière encore préférée au moins 0.55% de phospholipides, de manière préférée entre toutes au moins 0.60% de phospholipides (% en poids d'hydrolysat pur en poudre). Dans des modes de réalisation de l'invention, l'hydrolysat protéique comprend au moins 1% de phospholipides (% en poids d'hydrolysat pur en poudre). En particulier il comprend au moins 1.1% de phospholipides, de préférence au moins 1.2% de phospholipides, de préférence encore au moins 1.3% de phospholipides, de manière encore préférée au moins 1.4% de phospholipides, de manière préférée entre toutes au moins 1.5% de phospholipides (% en poids d'hydrolysat pur en poudre). Ledit hydrolysat contient un maximum de 3% de phospholipides, de préférence un maximum de 2.8% de phospholipides, de préférence encore un maximum de 2.5% de phospholipides, de manière encore préférée un maximum 2.3% de phospholipides, de manière préférée entre toutes un maximum de 2.1% de phospholipides (% en poids d'hydrolysat pur en poudre).

En particulier, les phospholipides représentent au moins 4%, de préférence au moins 4.5%, de préférence encore au moins 5%, de manière encore préférée au moins 5.5%, de manière encore préférée au moins 6%, de manière préférée entre toutes au moins 6.5% de la matière grasse totale de l'hydrolysat pur en poudre selon l'invention. Dans des modes de réalisation particuliers, les phospholipides représentent au moins 10%, de préférence au moins 11%, de préférence encore au moins 12%, de manière encore préférée au moins 14%, de manière préférée entre toutes au moins 15% de la matière grasse totale de l'hydrolysat pur en poudre selon l'invention. Notamment, les phospholipides représentent au maximum 70%, de préférence au maximum 65%, de préférence encore au maximum 60%, de manière encore préférée au maximum 55%, de manière préférée entre toutes au maximum 50% de la matière grasse totale de l'hydrolysat pur en poudre selon l'invention.

Selon un mode de réalisation préféré, l'hydrolysat de l'invention est riche en phosphatidylsérine (PS), phosphatidyléthanolamine (PE), phosphatidylcholine (PC) et phosphatidylinositol (PI), quatre phospholipides prédominants dans le cerveau.

Selon ce mode de réalisation, ledit hydrolysat contient notamment de la phosphatidylsérine dans une quantité d'au moins 0.1 mg, de préférence au moins 0.2 mg, de préférence encore au moins 0.3 mg, de manière encore préférée au moins 0.4 mg, de manière préférée entre toutes au moins 0.5 mg, par gramme d'hydrolysat pur en poudre.

Selon ce mode de réalisation, ledit hydrolysat contient notamment de la phosphatidylsérine dans une quantité inférieure ou égale à 5 mg, de préférence inférieure ou égale à 4 mg, de préférence encore inférieure ou égale à 3 mg, de manière encore préférée inférieure ou égale à 2.5 mg, de manière préférée entre toutes inférieure ou égale à 2 mg, par gramme d'hydrolysat pur en poudre.

Ledit hydrolysat contient également de la phosphatidyléthanolamine dans une quantité d'au moins 0.1 mg, de préférence au moins 0.2 mg, de préférence encore au moins 0.25 mg, de manière encore préférée au moins 0.3 mg, de manière préférée entre toutes au moins 0.35 mg, par gramme d'hydrolysat pur en poudre. Dans des modes de réalisation particuliers, ledit hydrolysat contient de la phosphatidyléthanolamine dans une quantité d'au moins 0.5 mg, de préférence au moins 0.8 mg, de préférence encore au moins 1.2 mg, de manière encore préférée au moins 1.4 mg, de manière préférée entre toutes au moins 1.5 mg, par gramme d'hydrolysat pur en poudre.

Ledit hydrolysat contient également de la phosphatidyléthanolamine dans une quantité inférieure ou égale à 7 mg, de préférence inférieure ou égale à 6 mg, de préférence encore inférieure ou égale à 5 mg, de manière encore préférée inférieure ou égale à 4.5 mg, de manière préférée entre toutes inférieure ou égale à 4 mg, par gramme d'hydrolysat pur en poudre.

Ledit hydrolysat contient encore de la phosphatidylcholine dans une quantité d'au moins 1 mg, de préférence au moins 1.5 mg, de préférence encore au moins 2 mg, de manière encore préférée au moins 2.5 mg, de manière encore préférée au moins 3 mg, de manière préférée entre toutes au moins 3.5 mg, par gramme d'hydrolysat pur en poudre. Dans des modes de réalisation particuliers, ledit hydrolysat contient de la phosphatidylcholine dans une quantité d'au moins 5 mg, de préférence au moins 6 mg, de préférence encore au moins 6.5 mg, de manière encore préférée au moins 7 mg, de manière préférée entre toutes au moins 7.5 mg, par gramme d'hydrolysat pur en poudre.

Ledit hydrolysat contient encore de la phosphatidylcholine dans une quantité inférieure ou égale à 20 mg, de préférence inférieure ou égale à 18 mg, de préférence encore inférieure ou égale à 16 mg, de manière encore préférée inférieure ou égale à 14 mg, de manière préférée entre toutes inférieure ou égale à 12.5 mg, par gramme d'hydrolysat pur en poudre.

Ledit hydrolysat contient aussi du phosphatidylinositol dans une quantité d'au moins 0.2 mg, de préférence au moins 0.3 mg, de préférence encore au moins 0.4 mg, de manière encore préférée au moins 0.45 mg, de manière préférée entre toutes au moins 0.5 mg, par gramme d'hydrolysat pur en poudre. Dans des modes de réalisation particuliers, ledit hydrolysat contient du phosphatidylinositol dans une quantité d'au moins 0.5 mg, de préférence au moins 0.7 mg, de préférence encore au moins 0.9 mg, de manière encore préférée au moins 1 mg, de manière préférée entre toutes au moins 1.1 mg, par gramme d'hydrolysat pur en poudre.

Ledit hydrolysat contient aussi du phosphatidylinositol dans une quantité inférieure ou égale à 5 mg, de préférence inférieure ou égale à 4 mg, de préférence encore inférieure ou égale à 3 mg, de manière encore préférée inférieure ou égale à 2.5 mg, de manière préférée entre toutes inférieure ou égale à 2.1 mg, par gramme d'hydrolysat pur en poudre.

Les acides gras polyinsaturés (AGPI) sont des acides gras dont la chaîne hydrocarbonée comprend au moins deux doubles liaisons. Parmi les différentes familles d'AGPI, deux familles, les AGPI de la série n-6 (ou oméga 6) et ceux de la série n-3 (ou oméga 3) sont d'un grand intérêt nutritionnel. Ils diffèrent par la position de la première double liaison. Ces deux familles dérivent de précurseurs métaboliques d'origine exclusivement végétale : l'acide alpha linolénique (n-3) (ALA) et l'acide linoléique (n-6) (LA) qui doivent donc obligatoirement être apportés par notre alimentation. Ce sont des acides gras indispensables. L'ALA se trouve essentiellement dans les graines et huiles de colza, de soja, de noix et de lin et le LA dans les graines et les huiles de tournesol, d'arachide, de maïs et de pépins de raisin. Une fois consommé, l'ALA est transformé en dérivés essentiels pour la santé : l'acide eicosapentaénoïque (EPA) et l'acide docosahexaénoïque (DHA). Les poissons marins, qui se nourrissent d'algues et de phytoplancton, sont une bonne source d'EPA et de DHA. Le LA quant à lui est métabolisé en acide arachidonique (AA), également essentiel pour la santé, qui est directement apporté par la consommation de produits provenant d'animaux terrestres (oeuf, viande).

Les AGPI présents dans le cerveau sont majoritairement l'AA et le DHA qui sont constitutifs des membranes cérébrales. L'incorporation des AGPI est la plus forte au cours du développement cérébral, tandis que dans le cerveau adulte, le DHA et l'AA ne s'accumulent plus, mais leurs niveaux sont maintenus par un « turnover » régulier qui compense leur utilisation. Les apports alimentaires sont susceptibles de faire varier ces niveaux.

De manière avantageuse, l'hydrolysat selon l'invention contient des acides gras polyinsaturés (AGPI), et en particulier une proportion importante d'oméga 3. En particulier, il comprend au moins 0.5%, de préférence au moins 1%, de préférence encore au moins 1.5%, de manière encore préférée au moins 2%, de manière préférée entre toutes au moins 2.5% d'acides gras polyinsaturés (AGPI), et notamment d'oméga 3 et d'oméga 6 (% en poids d'hydrolysat pur en poudre). De manière préférée, ledit hydrolysat contient un maximum de 5%, de préférence un maximum de 4.7%, de préférence encore un maximum de 4.5%, de manière encore préférée un maximum 4.2%, de manière préférée entre toutes un maximum de 4.0% d'acides gras polyinsaturés (AGPI), et notamment d'oméga 3 et d'oméga 6 (% en poids d'hydrolysat pur en poudre).

De préférence, l'hydrolysat selon l'invention comprend au moins 0.5%, de préférence au moins 0.7%, de préférence encore au moins 1%, de manière encore préférée au moins 1.5%, de manière préférée entre toutes au moins 2% d'oméga 3 (% en poids d'hydrolysat pur en poudre). De manière préférée, ledit hydrolysat contient un maximum de 4%, de préférence un maximum de 3.9%, de préférence encore un maximum de 3.8%, de manière encore préférée un maximum 3.7%, de manière préférée entre toutes un maximum de 3.5% d'oméga 3 (% en poids d'hydrolysat pur en poudre).

En particulier, les oméga 3 représentent au moins 15%, de préférence au moins 20%, de préférence encore au moins 25%, de manière encore préférée au moins 30% de la matière grasse totale de l'hydrolysat pur en poudre selon l'invention. Notamment, les oméga 3 représentent au maximum 50%, de préférence au maximum 45%, de préférence encore au maximum 40%, de manière encore préférée au maximum 35% de la matière grasse totale de l'hydrolysat pur en poudre selon l'invention.

En particulier, l'hydrolysat selon l'invention comprend au moins 0.05%, de préférence au moins 0.07%, de préférence encore au moins 0.1%, de manière encore préférée au moins 0.15%, de manière préférée entre toutes au moins 0.2% d'oméga 6 (% en poids d'hydrolysat pur en poudre). De manière préférée, ledit hydrolysat contient un maximum de 1.5%, de préférence un maximum de 1.4%, de préférence encore un maximum de 1.3%, de manière encore préférée un maximum 1.2%, de manière préférée entre toutes un maximum de 1.1% d'oméga 6 (% en poids d'hydrolysat pur en poudre).

En particulier, les oméga 6 représentent au moins 0.5%, de préférence au moins 0.75%, de préférence encore au moins 1%, de manière encore préférée au moins 2% de la matière grasse totale de l'hydrolysat pur en poudre selon l'invention. Notamment, les oméga 6 représentent au maximum 25%, de préférence au maximum 21%, de préférence encore au maximum 17%, de manière encore préférée au maximum 13% de la matière grasse totale de l'hydrolysat pur en poudre selon l'invention.

L'hydrolysat de l'invention est particulièrement avantageux du fait de la présence de DHA et d'EPA. L'hydrolysat protéique de l'invention comprend au moins 0.5% de DHA et EPA (en % d'hydrolysat pur en poudre). En particulier il comprend au moins 0.7% de DHA et EPA, de préférence au moins 0.9 % de DHA et EPA, de manière encore préférée au moins 1% de DHA et EPA, de manière préférée entre toutes au moins 1.5% de DHA et EPA (% en poids d'hydrolysat pur en poudre).

De manière préférée, ledit hydrolysat contient un maximum de 5% de DHA et EPA, de préférence un maximum de 4.5% de DHA et EPA, de préférence encore un maximum de 4% de DHA et EPA, de manière encore préférée un maximum 3.5% de DHA et EPA, de manière préférée entre toutes un maximum de 3.2% de DHA et EPA (% en poids d'hydrolysat pur en poudre).

En particulier, le DHA et l'EPA représentent au moins 10%, de préférence au moins 15%, de préférence encore au moins 20%, de manière encore préférée au moins 22% de la matière grasse totale de l'hydrolysat pur en poudre selon l'invention. Notamment, le DHA et l'EPA représentent au maximum 40%, de préférence au maximum 35%, de préférence encore au maximum 30%, de manière encore préférée au maximum 27% de la matière grasse totale de l'hydrolysat pur en poudre selon l'invention.

Selon un autre mode de réalisation préféré, il contient notamment du DHA dans une quantité d'au moins 2 mg, de préférence au moins 3 mg, de préférence encore au moins 5 mg, de manière encore préférée au moins 7 mg, de manière préférée entre toutes au moins 9 mg, par gramme d'hydrolysat pur en poudre.

Ledit hydrolysat contient préférentiellement du DHA dans une quantité inférieure ou égale à 30 mg, de préférence inférieure ou égale à 27 mg, de préférence encore inférieure ou égale à 25 mg, de manière encore préférée inférieure ou égale à 23 mg, de manière préférée entre toutes inférieure ou égale à 20 mg, par gramme d'hydrolysat pur en poudre.

En particulier, le DHA représente au moins 5%, de préférence au moins 5.3%, de préférence encore au moins 5.7%, de manière encore préférée au moins 5.9%, de manière préférée entre toutes au moins 6% de la matière grasse totale de l'hydrolysat pur en poudre selon l'invention. Notamment, le DHA représente au maximum 30%, de préférence au maximum 27%, de préférence encore au maximum 25%, de manière encore préférée au maximum 22%, de manière préférée entre toutes au maximum 20% de la matière grasse totale de l'hydrolysat pur en poudre selon l'invention.

Associé aux peptides, le DHA, présent mais non prépondérant, produit avantageusement des effets bénéfiques.

Selon un autre mode de réalisation préféré, ledit hydrolysat protéique contient également de l'EPA dans une quantité d'au moins 0.5 mg, de préférence au moins 1 mg, de préférence encore au moins 2 mg, de manière encore préférée au moins 3 mg, de manière préférée entre toutes au moins 4 mg, par gramme d'hydrolysat pur en poudre.

Ledit hydrolysat contient préférentiellement de l'EPA dans une quantité inférieure ou égale à 25 mg, de préférence inférieure ou égale à 24 mg, de préférence inférieure ou égale à 23 mg, de préférence encore inférieure ou égale à 22 mg, de manière encore préférée inférieure ou égale à 21 mg, de manière préférée entre toutes inférieure ou égale à 20 mg, par gramme d'hydrolysat pur en poudre.

En particulier, l'EPA représente au moins 1%, de préférence au moins 2%, de préférence encore au moins 4%, de manière encore préférée au moins 6% de la matière grasse totale de l'hydrolysat pur en poudre selon l'invention. Notamment, l'EPA représente au maximum 40%, de préférence au maximum 35%, de préférence encore au maximum 30%, de manière encore préférée au maximum 25% de la matière grasse totale de l'hydrolysat pur en poudre selon l'invention.

De manière particulièrement avantageuse, une proportion d'oméga 3 de l'hydrolysat selon l'invention, et notamment le DHA et EPA, est liée aux phospholipides.

En particulier, l'hydrolysat selon l'invention comprend au moins 0.3%, de préférence au moins 0.35%, de préférence encore au moins 0.4%, de manière encore préférée au moins 0.45%, de manière préférée entre toutes au moins 0.5% d'oméga 3 liés aux phospholipides. De manière préférée, ledit hydrolysat contient un maximum de 1%, de préférence un maximum de 0.95%, de préférence encore un maximum de 0.9%, de manière encore préférée un maximum 0.85%, de manière préférée entre toutes un maximum de 0.8% d'oméga 3 liés aux phospholipides.

Plus particulièrement, l'hydrolysat selon l'invention comprend au moins 0.15%, de préférence au moins 0.20%, de préférence encore au moins 0.25%, de manière encore préférée au moins 0.30%, de manière préférée entre toutes au moins 0.35% de DHA lié aux phospholipides. De manière préférée, ledit hydrolysat contient un maximum de 1%, de préférence un maximum de 0.9%, de préférence encore un maximum de 0.8%, de manière encore préférée un maximum 0.7%, de manière préférée entre toutes un maximum de 0,65% de DHA lié aux phospholipides.

Plus particulièrement, l'hydrolysat selon l'invention comprend au moins 0.050%, de préférence au moins 0.055%, de préférence encore au moins 0.060%, de manière encore préférée au moins 0.070%, de manière préférée entre toutes au moins 0.080% d'EPA lié aux phospholipides. De manière préférée, ledit hydrolysat contient un maximum de 0.2%, de préférence un maximum de 0.18%, de préférence encore un maximum de 0.15%, de manière encore préférée un maximum 0.13%, de manière préférée entre toutes un maximum de 0.11 % d'EPA lié aux phospholipides.

En particulier, l'hydrolysat selon l'invention comprend au moins 0.005%, de préférence au moins 0.01%, de préférence encore au moins 0.015%, de manière encore préférée au moins 0.02%, de manière préférée entre toutes au moins 0.025% d'oméga 6 liés aux phospholipides. De manière préférée, ledit hydrolysat contient un maximum de 0.5%, de préférence un maximum de 0.4%, de préférence encore un maximum de 0.3%, de manière encore préférée un maximum 0.2%, de manière préférée entre toutes un maximum de 0.15% d'oméga 6 liés aux phospholipides.

Les « lipides neutres » ou « graisses neutres » ou « lipides simples », constitués essentiellement de triglycérides (ou triacylglycérols), représentent la principale forme de stockage des graisses dans les cellules adipeuses. Selon un mode de réalisation particulier, l'hydrolysat selon l'invention comprend au moins 0.1%, de préférence au moins 0.2%, de préférence encore au moins 0.3%, de manière encore préférée au moins 0.4%, de manière préférée entre toutes au moins 0.5% de lipides neutres, de préférence des triglycérides (% en poids d'hydrolysat pur en poudre). De manière préférée, ledit hydrolysat contient un maximum de 15%, de préférence un maximum de 14%, de préférence encore un maximum de 12%, de manière encore préférée un maximum 10% de lipides neutres, de préférence des triglycérides (% en poids d'hydrolysat pur en poudre).

Selon un mode de réalisation particulier, l'hydrolysat selon l'invention comprend au moins 0.05 mg, de préférence au moins 0.10 mg, de préférence encore au moins 0.15 mg, de manière encore préférée au moins 0.20 mg de plasmalogènes par gramme d'hydrolysat pur en poudre. De manière préférée, ledit hydrolysat contient un maximum de 1.0 mg, de préférence un maximum de 0.8 mg, de préférence encore un maximum de 0.7 mg, de manière encore préférée un maximum 0.5 mg de plasmalogènes par gramme d'hydrolysat pur en poudre.

L'hydrolysat peut se présenter sous n'importe quelle forme, et notamment sous forme liquide ou sous forme de poudre. Selon un mode de réalisation particulier, l'hydrolysat selon l'invention se présente sous la forme d'une poudre. Selon ce mode de réalisation particulier, l'hydrolysat comprend de préférence au moins 1%, de préférence au moins 2% d'humidité, de préférence encore au moins 3%, de manière encore préférée au moins 4% d'humidité (% en poids d'hydrolysat pur en poudre). En outre, ledit hydrolysat comprend au maximum 15%, de préférence au maximum de 12%, de préférence encore au maximum 10%, de manière encore préférée au maximum 9%, de manière préférée entre toutes au maximum 8% d'humidité (% en poids d'hydrolysat pur en poudre).

L'hydrolysat contient des matières minérales. En particulier, l'hydrolysat peut comprendre de préférence au moins 5%, de préférence au moins 6%, de préférence encore au moins 7%, de manière encore préférée au moins 8%, de manière préférée entre toutes au moins 8,5% de matières minérales (% en poids d'hydrolysat pur en poudre). En outre, ledit hydrolysat comprend au maximum 20%, de préférence au maximum de 15%, de préférence encore au maximum 12%, de manière encore préférée au maximum 11%, de manière préférée entre toutes au maximum 10% de matières minérales (% en poids d'hydrolysat pur en poudre).

En particulier, l'hydrolysat comprend de préférence entre 2 mg et 8 mg, de préférence encore entre 2.3 mg et 7.8 mg, de préférence encore entre 2.5 mg et 7.5 mg, de manière encore préférée entre 2.8 mg et 7.3 mg, de manière préférée entre toutes entre 3 mg et 7 mg de sélénium par kilogramme d'hydrolysat pur en poudre selon l'invention.

En particulier, l'hydrolysat comprend de préférence entre 12 mg et 60 mg, de préférence encore entre 15 mg et 55 mg, de préférence encore entre 18 mg et 50 mg, de manière encore préférée entre 20 mg et 48 mg, de manière préférée entre toutes entre 22 mg et 45 mg de zinc par kilogramme d'hydrolysat pur en poudre selon l'invention.

En particulier, l'hydrolysat comprend de préférence entre 400 mg et 1500 mg, de préférence encore entre 450 mg et 1450 mg, de préférence encore entre 500 mg et 1400 mg, de manière encore préférée entre 550 mg et 1350 mg, de manière préférée entre toutes entre 600 mg et 1300 mg de calcium par kilogramme d'hydrolysat pur en poudre selon l'invention.

En particulier, l'hydrolysat comprend de préférence entre 2000 mg et 8000 mg, de préférence encore entre 2150 mg et 7500 mg, de préférence encore entre 2300 mg et 7000 mg, de manière encore préférée entre 2500 mg et 6500 mg, de manière préférée entre toutes entre 2750 mg et 6000 mg de phosphore par kilogramme d'hydrolysat pur en poudre selon l'invention.

L'hydrolysat comprend de préférence entre 0.01 mg et 1 mg, de préférence encore entre 0.03 mg et 0.7 mg, de préférence encore entre 0.05 mg et 0.5 mg, de manière encore préférée entre 0.07 mg et 0.4 mg, de manière préférée entre toutes entre 0.1 mg et 0.2 mg de vitamine B12 par 100 grammes d'hydrolysat pur en poudre selon l'invention.

L'hydrolysat comprend de préférence entre 0.004 mg et 0.06 mg, de préférence encore entre 0.005 mg et 0.05 mg, de préférence encore entre 0.006 mg et 0.04 mg, de manière encore préférée entre 0.007 mg et 0.03 mg, de manière préférée entre toutes entre 0.008 mg et 0.02 mg de vitamine D3 par 100 grammes d'hydrolysat pur en poudre selon l'invention.

Selon un mode de réalisation préféré, la source protéique utilisée pour l'invention est au moins issue de têtes de poissons bleus. Selon un autre mode de réalisation préféré, ladite source protéique est issue de sardines. Selon un mode de réalisation préféré entre tous, ladite source protéique est au moins issue de têtes de sardines.

On entend par « sardine » un petit poisson pélagique qui se nourrit de plancton, d'oeufs et de larves de crustacés. Son aire de répartition géographique s'étend en particulier de la partie centrale de la mer du Nord jusqu'au cap Blanc en Mauritanie. L'espèce est également abondante en Méditerranée jusqu'à la mer Noire. Poisson gras dont la teneur en lipides varie de 3,2 à 15% il est intéressant pour ses qualités nutritionnelles. En effet, la sardine est l'un des poissons les plus riches en lipides et spécifiquement en acides gras de la famille des omégas 3 (20 à 30 % des acides gras totaux) avec un rapport acides gras insaturés/acides gras saturés, proche de 2. La sardine est pauvre en glucides (<0,1% par rapport au poids frais) mais est riche en protéines de très bonne valeur nutritionnelle, source d'acides aminés indispensables, 100g de sardine suffisent à couvrir 100% des besoins quotidiens en acides aminés. Outre la présence de protéines de qualité, d'acides gras polyinsaturés (AGPI) à longue chaîne (EPA-DHA), phospholipides..., la sardine renferme aussi des minéraux tels que fer et zinc ; elle contient peu de sodium mais est riche en calcium, magnésium, potassium, sélénium. Elle contient des vitamines A, D, B3 (nicotinamide), B6 (pyridoxine), B12 (cobalamine) et E (d-tocophérol). Une portion de 150g de sardine couvre les besoins journaliers en vitamines D et E d'un individu humain « standard ». Elle est aussi très intéressante pour ses apports en coenzyme Q10. La sardine est un poisson de début de chaîne trophique, qui présente l'avantage de contenir des niveaux faibles en contaminants de type métaux lourds, comme le méthyl-mercure, ou PCB.

Selon un autre mode de réalisation, la source protéique utilisée pour l'invention comprend des têtes de poissons bleus, de préférence ladite source protéique est issue de têtes seules. Dans un mode de réalisation alternatif, la source protéique utilisée pour l'invention comprend des têtes et des viscères de poissons bleus, de préférence ladite source protéique est issue de têtes et comprend en outre des viscères, de préférence encore ladite source protéique utilisée pour l'invention est issue de têtes et de viscères. Dans un mode de réalisation particulier, les viscères représentent moins de 30% en poids de la source protéique, de préférence moins de 20% en poids, de préférence encore moins de 15% en poids. Si la source protéique comprend des viscères, les enzymes endogènes desdites viscères sont préférablement inactivées. Ceci permet d'empêcher une autolyse de la source protéique et d'obtenir un hydrolysat protéique dont les caractéristiques physicochimiques sont standardisées. De manière avantageuse, l'hydrolysat de l'invention comprenant des têtes et/ou viscères permet de valoriser des coproduits marins, et d'assurer à terme une pêche durable. Ces coproduits, récupérés lors de la transformation des produits de la mer (comprenant notamment le filetage, l'éviscération, l'étêtage etc.), ne sont classiquement pas consommés dans l'alimentation humaine. L'obtention de ces hydrolysats constitue ainsi une approche d'un intérêt stratégique majeur pour réhabiliter la fraction protéique des coproduits marins.

Les poissons bleus, et notamment les sardines, sont donc une excellente source d'acide gras polyinsaturés (AGPI), oméga-3 et oméga-6, et en particulier une excellente source de DHA. Toutefois la quantité et la nature des acides gras peuvent varier en fonction du régime alimentaire desdits poissons. La teneur en DHA dans les hydrolysats peut donc être soumise à certaines variations en fonction notamment de critères tels que la saison ou le lieu de pêche. Ainsi, dans un mode de réalisation particulier, l'hydrolysat protéique de l'invention est complémenté en DHA. En d'autres termes, du DHA exogène ou une autre source que l'hydrolysat, comprenant du DHA, peut être ajoutée audit hydrolysat selon l'invention. L'hydrolysat peut notamment être complémenté en DHA dans un ratio hydrolysat : DHA compris entre 1 :5 et 5 :1, de préférence compris entre 1 :2 et 2 :1.

### Procédé d'hydrolyse

Parmi les procédés biotechnologiques offrant un champ très dynamique de recherche et d'applications industrielles, l'hydrolyse enzymatique des protéines permet de générer une fraction soluble appelée « hydrolysat ». Composé majoritairement de peptides et d'acides aminés libres, l'hydrolysat de l'invention se caractérise par de nouvelles propriétés fonctionnelles, nutritionnelles et biologiques, et trouve des applications en alimentation humaine et animale et dans le domaine des nutraceutiques et des médicaments. Il est connu de l'Homme du métier que la sélection des matières premières et des conditions de la réaction d'hydrolyse sont déterminantes pour l'obtention d'un d'hydrolysat présentant un profil peptidique et moléculaire particulier. Par ailleurs, les propriétés biochimiques de l'hydrolysat conditionnent son activité biologique. Ainsi, deux hydrolysats issus d'une même source protéique mais présentant des profils peptidiques différents ou une teneur en lipides différente pourront ne pas présenter le même niveau d'activité, voire même avoir des activités biologiques différentes.

On entend par « **source protéique** » ou « **matériel protéique** » ou « **fraction protéique** » ou « **substrat protéique** » un matériel d'origine naturelle, de préférence d'origine marine, de préférence encore issu de poissons bleus, constitué en partie de protéines et/ou peptides et/ou acides aminés.

Le procédé d'hydrolyse enzymatique développé dans le cadre de la présente invention est un procédé doux, sans solvant, qui se déroule en milieu aqueux : les coproduits de poissons bleus, notamment au moins les têtes de poisson bleus, et de préférence au moins les têtes de sardines, sont mis en présence d'une enzyme unique ou d'un mélange d'enzymes, de type endo- et/ou exopeptidase(s) dans un milieu contenant de l'eau, dont le pH et la température ont été ajustés afin de correspondre à l'optimum d'activité des enzymes utilisées (selon les recommandations des fabricants). Les enzymes déstructurent les protéines contenues dans les coproduits en rompant les liaisons peptidiques entre les acides aminés constitutifs des protéines. Chaque enzyme ayant une action sélective, le choix de(s) enzyme(s) permet de cibler de manière spécifique les liaisons peptidiques à couper. Puis, les hydrolysats subissent des étapes de séparation et de séchage pour obtenir une poudre sous une forme stabilisée.

Par « **enzyme endopeptidase** » ou « **endoprotéase** » on entend une enzyme protéolytique capable de rompre les liaisons peptidiques à l'intérieur d'un peptide et/ou d'une protéine, c'est-à-dire les liaisons peptidiques entre les acides aminés non terminaux. Parmi les endoprotéases, on trouve notamment :
- les endopeptidases à sérine dont la trypsine qui coupe après les acides aminés arginine ou lysine, sauf si cette dernière est suivie par une proline ; la chymotrypsine qui coupe après les acides aminés phénylalanine, tryptophane ou tyrosine, à moins qu'ils ne soient suivis d'une proline ; l'élastase qui coupe après les résidus alanine, glycine, serine ou valine, à moins qu'ils ne soient suivis d'une proline ; la subtilisine, qui catalyse l'hydrolyse des protéines avec une faible spécificité en matière de liaison peptidique et qui agit préférentiellement à proximité d'un grand résidu d'acide aminé non chargé ;

- les endopeptidases à cystéine telles que la papaïne et la ficine qui exigent la présence d'un groupe -SH libre dans leur site actif pour exercer une action protéolytique ;
- les endopeptidases à acide aspartique telles que la pepsine qui contiennent au niveau du site actif un résidu acide aspartique impliqué dans la catalyse. La pepsine coupe avant les résidus leucine, phénylalanine, tryptophane ou tyrosine, sauf s'ils sont précédés d'une proline.

Par « **enzyme exopeptidase** » ou « **exoprotéase** » on entend une enzyme protéolytique capable de rompre les liaisons peptidiques à l'extrémité N-terminale ou C-terminale d'un peptide et/ou d'une protéine. On parlera respectivement d'aminopeptidase ou de carboxypeptidase. Les exopeptidases permettent de générer dans l'hydrolysat des monomères, c'est-à-dire des acides aminés libres.

Selon un mode de réalisation particulièrement préféré, l'hydrolysat protéique de l'invention est obtenu par un procédé d'hydrolyse enzymatique comprenant les étapes de :
a. fourniture d'une source protéique ;
b. broyage de ladite source protéique ;
c. optionnellement, ajustement du pH ;
d. ajout d'au moins une enzyme d'hydrolyse ;
e. chauffage ;
f. séparation ;
g. séchage.

En fonction du substrat protéique de départ, du degré d'hydrolyse souhaité, notamment supérieur à 10%, mais aussi de la teneur spécifique en peptides de tailles données, notamment, les peptides d'une taille inférieure à 5000 Da, ou inférieure à 2000 Da, ou inférieure à 1000 Da ou encore inférieure à 500 Da, l'Homme du métier saura adapter de manière spécifique les conditions de réaction. L'étape d'hydrolyse enzymatique nécessite de déterminer les enzymes à utiliser et d'ajuster divers paramètres tels que le ratio enzyme/substrat, le temps d'hydrolyse, la vitesse d'agitation, le pH et la température.

Selon un mode particulier, à l'étape (a), la source protéique est préférentiellement constituée de poissons bleus, notamment de sardines, de préférence au moins de têtes de poissons bleus et de manière préférée entre toutes, au moins de têtes de sardines.

A l'étape (b), le broyage de la source protéique permet avantageusement d'obtenir la taille de particule appropriée. Le broyage doit être le plus fin possible pour favoriser l'action des enzymes. Idéalement, toutes les particules doivent avoir une taille proche de 5 mm, de préférence proche de 3 mm. L'étape de broyage peut être effectuée sur une source protéique sèche ou humide. L'étape de broyage nécessite ainsi d'ajuster la durée et la vitesse en fonction de l'instrument employé et de la nature sèche ou humide du matériau à broyer, ce dont est capable l'homme du métier dans l'exercice de son travail de routine. L'addition d'eau nécessite d'ajuster la vitesse, la durée, le ratio eau/solide en fonction notamment de l'instrument à employer, tout ajustement là encore à la portée de l'homme du métier. De préférence, une certaine masse d'eau est ajoutée à la source protéique. Elle est préférablement égale à 0,1 à 1 fois la masse de ladite source protéique et ne dépasse pas cette quantité.

Selon un mode réalisation, le précédé d'hydrolyse comprend une étape optionnelle d'ajout d'antioxydant. Cet ajout optionnel peut avoir lieu à diverses étapes du procédé, notamment avant ou après broyage (étape b) mais aussi avant le séchage (étape g). On entend par antioxydant un composé chimique ou naturel capable d'inhiber les réactions d'oxydation et de prévenir notamment la formation de radicaux libres. L'homme du métier saura adapter la nature et la quantité d'antioxydant à mettre en oeuvre afin d'adapter au mieux la réaction d'hydrolyse, en fonction notamment de la source protéique utilisée.

L'étape optionnelle c) d'ajustement du pH permet d'adapter, au besoin, le pH de la source protéique broyée. Il est en effet important que le pH de ce broyat corresponde au pH optimal de fonctionnement de ladite enzyme d'hydrolyse. L'homme du métier connaît les moyens d'adapter le pH dudit broyat, notamment en utilisant des bases et/ou acides adaptés aux procédés agroalimentaires, notamment une base telle que bicarbonate de sodium permettra d'augmenter le pH dudit broyat, tandis qu'un acide tel que l'acide citrique ou l'acide acétique permettra de le faire baisser.

En particulier à l'étape (d), ladite au moins une enzyme utilisée contient au moins une endoprotéase et/ou au moins une exoprotéase, de préférence au moins une endoprotéase, comme par exemple une endopeptidase à sérine, une endopeptidase à acide aspartique et/ou une endopeptidase à cystéine, de préférence une endopeptidase à sérine. De manière préférée, l'endopeptidase de l'invention est une endopeptidase alcaline et de préférence une endopeptidase d'origine microbienne.

Selon un mode de réalisation préféré de l'étape (d), ladite au moins une enzyme utilisée contient au moins une alcalase, en particulier l'alcalase 2.4L comme illustré dans les exemples.

Avantageusement, le chauffage selon l'étape (e) est réalisé pendant 1 à 20 heures, de préférence entre 2 et 12 heures, à une température comprise entre 25°C et 70°C, de préférence entre 40° C et 60° C, et de préférence autour de 55°C. Comme l'homme du métier le sait bien, l'étape de chauffage peut comprendre plusieurs paliers de température, notamment en vue de désactiver les enzymes par chauffage, typiquement entre 80°C et 105°C pendant par exemple 15 à 40 minutes.

Selon un mode particulier, le mélange est préfiltré sur tamis afin de retirer les particules solides (en particulier les arêtes).

Puis le milieu réactionnel est séparé, selon l'étape (f), en utilisant n'importe quelle technique de séparation connue adaptée, telle que la centrifugation ou la décantation. De préférence, le milieu réactionnel est séparé par centrifugation, et notamment centrifugation verticale et/ou horizontale. L'étape de séparation nécessite d'ajuster des paramètres tels que la vitesse, la température, la durée et le pH, ce qui relève de l'application en routine des connaissances générales de l'homme du métier. Cette étape permet avantageusement de récupérer la fraction soluble, contenant des peptides solubles, phospholipides et DHA/EPA, et d'éliminer les protéines insolubles ainsi qu'une partie de la matière grasse, contenant majoritairement des lipides neutres, et en particulier des triglycérides.

La phase aqueuse obtenue après séparation est ensuite séchée (étape g) en utilisant n'importe quelle technique de séchage connue adaptée, telle que le séchage par sublimation (lyophilisation) ou le séchage par évaporation, notamment par atomisation ou dessication. De manière optionnelle, il est possible de procéder préalablement à l'étape de séchage, à une étape de concentration. L'Homme du métier saura choisir la technique de séchage la plus adaptée et optimiser les conditions de séchage en fonction de l'utilisation qui sera faite de l'hydrolysat protéique de l'invention. En particulier, il pourra choisir d'utiliser au moins un support de séchage. Le terme « support de séchage » fait référence à n'importe quel composé conventionnel pour la mise en oeuvre d'une étape de séchage dans le domaine technique de l'invention. Par exemple, un support de séchage approprié peut être choisi parmi les protéines microbiennes (e.g., levures), les protéines laitières (e.g., caséinates), les amidons hydrolysés (e.g., maltodextrines), les amidons modifiés (e.g., octenyl succinate amidon), les cyclodextrines, les gommes (e.g., gomme arabique), les fibres (e.g., fibres de cellulose), et leurs combinaisons.

En particulier, il est essentiel, pour obtenir l'hydrolysat protéique de l'invention, c'est-à-dire un hydrolysat riche en peptides, et comprenant des phospholipides et DHA/EPA, (1) de réaliser une hydrolyse protéique avec un degré d'hydrolyse d'au moins 10%, et (2) de procéder à une séparation des phases.

Selon un mode de réalisation particulier, le procédé d'hydrolyse enzymatique comprend en outre une étape de supplémentation en DHA. L'hydrolysat protéique de l'invention est alors obtenu par un procédé d'hydrolyse enzymatique comprenant les étapes de :
a. fourniture d'une source protéique ;
b. broyage de ladite source protéique ;
c. optionnellement, ajustement du pH ;
d. ajout d'au moins une enzyme d'hydrolyse ;
e. chauffage ;
f. séparation ;
f' addition de DHA ;
g. séchage.

Dans un mode de réalisation alternatif, l'étape d'addition de DHA (étape (f')) est réalisée après l'étape de séchage (g). Le DHA peut notamment être du DHA « pur » ou un ingrédient en contenant en quantité significative. A titre d'exemple non limitatif, ledit ingrédient contenant une quantité significative de DHA peut être une huile de krill.

Ainsi, un autre aspect de l'invention concerne un procédé de préparation d'un hydrolysat protéique selon l'invention comprenant les étapes de :
a. fourniture d'une source protéique ;
b. broyage de ladite source protéique ;
c. optionnellement, ajustement du pH ;
d. ajout d'au moins une enzyme d'hydrolyse ;
e. chauffage ;
f. séparation ;
g. séchage.

Dans un mode de réalisation particulier, ce procédé comprend en outre une étape d'ajout d'antioxydant qui peut notamment être réalisée avant ou après l'étape de broyage (b), ou avant l'étape de séchage (g).

Dans un autre mode de réalisation particulier, ce procédé comprend en outre une étape (f') de supplémentation en DHA qui est réalisée après l'étape (f) de séparation et avant l'étape (g) de séchage. Dans un mode de réalisation alternatif, ce procédé comprend une étape (g') de supplémentation en DHA qui est réalisée après l'étape (g) de séchage.

Dans un autre mode de réalisation particulier, ce procédé comprend en outre une étape d'ajout d'antioxydant et une étape de supplémentation en DHA. Ladite étape d'ajout d'antioxydant pouvant notamment être réalisée avant ou après l'étape de broyage (b), ou avant l'étape de séchage (g) ; et ladite étape de supplémentation en DHA pouvant être réalisée après l'étape de séparation (f) et avant l'étape de séchage (g), ou réalisée après l'étape de séchage (g).

### Composition alimentaire et/ou pharmaceutique

L'hydrolysat protéique de l'invention (également appelé ici hydrolysat pur en poudre) est donc un hydrolysat riche en peptides, et contient des phospholipides, du DHA et de l'EPA. Ainsi, quand il est administré en quantité suffisante, l'hydrolysat de l'invention procure avantageusement des effets bénéfiques pour la santé.

### Composition alimentaire

Selon un autre aspect, l'invention porte sur une composition alimentaire comprenant au moins un hydrolysat protéique selon l'invention et tel que défini ci-dessus, caractérisée en ce qu'il s'agit d'un aliment complet ou d'un complément alimentaire, notamment un aliment fonctionnel ou nutraceutique.

Selon un premier mode de réalisation préféré, la composition alimentaire de l'invention est un complément alimentaire, notamment un aliment fonctionnel ou nutraceutique pour l'Homme.

On entend par « Homme » un individu humain de sexe masculin ou féminin, de préférence adulte, en particulier âgé(e) et à risque de développer des troubles cognitifs liés au vieillissement.

De manière avantageuse on distingue le sujet en fonction de l'utilisation thérapeutique ou prophylactique de ladite composition alimentaire. Ainsi, dans le cadre d'une utilisation thérapeutique ledit sujet est un adulte de préférence âgé d'au moins 60 ans ou plus. Dans le cadre d'une utilisation prophylactique, notamment pour prévenir les risques de déclin cognitif, ledit sujet est un adulte de préférence âgé d'au moins 50 ans ou plus.

L'expression « **complément alimentaire** » désigne un produit qui est destiné à être ingéré en plus du régime alimentaire normal. Il s'agit notamment d'une composition concentrée en nutriments, notamment peptides, phospholipides, DHA, vitamines et/ou minéraux qui comprend des substances à but nutritionnel ou physiologique. On entend par « **aliment fonctionnel** » un aliment semblable en apparence aux aliments conventionnels, faisant partie de l'alimentation normale, et procurant des bienfaits physiologiques démontrés et/ou réduisant le risque de maladie chronique au-delà des fonctions nutritionnelles de base. On entend par « **aliment nutraceutique** » un aliment présenté sous forme de pilules, de poudres ou sous d'autres formes médicinales qui ne sont pas généralement associées à des aliments. L'aliment nutraceutique présente un effet physiologique bénéfique ou une protection contre les maladies chroniques.

Selon ce mode de réalisation, ladite composition alimentaire comprenant au moins un hydrolysat protéique selon l'invention est destinée à être consommée par voie orale. Elle peut donc être bue et/ou ingérée. De façon avantageuse, la composition alimentaire de l'invention peut se présenter sous la forme d'une poudre. Ladite composition alimentaire sous forme de poudre peut ainsi être conditionnée dans des capsules ou des gélules, notamment des capsules ou des gélules à dissoudre et/ou à avaler.

Ainsi, ledit hydrolysat protéique est présent dans la composition alimentaire de l'invention en une quantité suffisante pour permettre, sur la base d'une dose quotidienne, un apport journalier dudit hydrolysat allant de 0.2 grammes (gr) à 7 gr, de préférence de 0.2 gr à 3 gr et de préférence encore de 0.5 gr à 3 gr. Ces doses sont données à titre indicatif pour un individu humain d'un poids de 60 kg. L'Homme du métier saura adapter ces doses en fonction notamment de l'âge et/ou du poids et/ou du régime alimentaire et/ou de l'état de santé général dudit individu.

Un autre objet de l'invention concerne un produit alimentaire caractérisé en ce qu'il comprend comme ingrédient une composition alimentaire telle que définie ci-dessus.

Ainsi, dans un mode de réalisation particulièrement avantageux, la composition de l'invention peut être incorporée à des aliments et/ou boissons. A titre d'exemple non limitatif, la composition de l'invention peut être intégrée lors de la préparation de jus de fruit et/ou de légumes, de produits laitiers fermentés ou non fermentés, comme par exemple des yaourts ou des yaourts à boire, des fromages, mais aussi des glaces ou crèmes glacées ; elle peut être également incorporée lors de la préparation de biscuits, barres alimentaires, dragées, pastilles, bonbons ou chewing-gum, etc.

Selon un second mode de réalisation préféré, la composition alimentaire de l'invention est un aliment pour animaux de compagnie, de préférence un aliment pour chiens ou chats.

Selon ce mode de réalisation, la composition alimentaire à destination des animaux de compagnie est de préférence un aliment complet.

Les termes « animal de compagnie », « animal domestique » et « animal » sont synonymes et désignent tout animal domestiqué comprenant, de manière non restrictive, des chats, des chiens, des lapins, des cobayes, des furets, des hamsters, des souris, des gerbilles des oiseaux, des chevaux, des vaches, des chèvres, des moutons, des ânes, des cochons et autres, et de préférence, des chats ou des chiens. De préférence, l'animal est adulte, en particulier âgé et à risque de développer des troubles cognitifs liés au vieillissement. Selon un mode de réalisation particulier, l'animal est un chien, dont l'âge est de préférence supérieur à 6 ans, de préférence encore supérieur à 7 ans.

Par « **aliment pour animaux de compagnie** » on désigne tout aliment qui peut se présenter sous une forme quelconque, solide, sèche, humide, semi-humide ou leurs combinaisons. Les friandises sont incluses dans les aliments pour animaux de compagnie.

On entend par « **aliment complet** » un aliment qui contient tous les nutriments connus requis pour le receveur ou le consommateur prévu de l'aliment, en quantités et proportions appropriées sur la base, par exemple, de recommandations des autorités reconnues ou compétentes dans le domaine de l'alimentation de l'animal de compagnie considéré (espèce/race/type/âge/...). Ces aliments constituent la seule source d'apports nutritionnels pour répondre aux besoins des animaux de compagnie, sans l'addition de sources alimentaires supplémentaires. Les aliments pour animaux domestiques équilibrés sur le plan nutritionnel sont largement connus et utilisés dans l'art.

Il existe trois catégories ou classes principales d'aliments complets pour animaux de compagnie, en fonction de leur teneur en humidité qui est faible ou moyenne ou élevée :
- les produits secs ou présentant une faible humidité (ayant moins d'environ 14 % d'humidité), tels que les croquettes ; ils sont généralement hautement nutritifs, peuvent être conditionnés par exemple dans des sacs ou des boîtes et sont hautement appropriés pour le stockage et l'utilisation ;
- les produits en boîte ou pochons ou humides ou présentant une teneur élevée en humidité (ayant plus d'environ 50 % d'humidité), tels que les produits « X avec des morceaux » : habituellement, des produits à teneur élevée en viande ;
- les produits semi-humides ou semi-secs ou secs et tendres ou humides et tendres ou des produits à teneur en humidité moyenne ou intermédiaire (ayant d'environ 14 % à environ 50 % d'humidité), tels que des pâtées : généralement conditionnées dans des sacs ou boîtes appropriés.

Le terme « **croquettes** » tel qu'il est utilisé ici désigne des fragments ou des morceaux particulaires formés par un processus d'agglomération ou d'extrusion. Habituellement, les croquettes sont produites pour donner un aliment pour animaux de compagnie sec et semi-humide, de préférence un aliment sec pour animaux de compagnie. Les morceaux peuvent varier en tailles et formes en fonction du processus ou de l'équipement. Par exemple, les croquettes peuvent avoir des formes sphériques, cylindriques, ovales ou similaires. Elles peuvent avoir une dimension maximale inférieure à environ 2 cm par exemple.

L'expression « **produits X avec des morceaux** » désigne ici tous les aliments comestibles comprenant des morceaux dans une préparation (ladite préparation étant la « préparation X »). Les exemples classiques de ceux-ci sont les produits avec des morceaux en gelée, les produits avec des morceaux en sauce, et autres. Cette catégorie de produits « X avec des morceaux » englobe également les formes comestibles autres que des morceaux qui peuvent être contenues dans la préparation X telles qu'une gelée, une sauce, et autres. Par exemple, d'autres formes que les morceaux peuvent être des produits en tranches, des produits râpés, etc.

Le terme « **pâtée** » tel qu'il est utilisé ici désigne des aliments comestibles obtenus sous la forme de produits humides et comprennent les terrines, les pâtés, les mousses, et autres.

Le terme « **friandise** » (ou « **biscuit** ») désigne tout aliment qui est conçu pour être donné par son propriétaire à un animal de compagnie, de préférence à un moment hors des repas, pour contribuer, favoriser ou maintenir un processus d'attachement entre un animal de compagnie et son propriétaire. Les friandises ou biscuits ne sont habituellement pas aptes à procurer un « aliment complet ». Les exemples de friandises pour chiens sont les os. Les exemples de friandises pour chats sont des coussinets à croquer et des bâtonnets à mâcher.

En particulier, l'hydrolysat protéique de l'invention peut être ajouté à ladite composition alimentaire pour animaux de compagnie par enrobage ou par inclusion, de préférence par inclusion.

On désigne par « **enrobage** » le fait de procéder à un dépôt topique de l'hydrolysat de l'invention, c'est-à-dire que le dépôt se fait sur la surface de l'aliment pour animaux de compagnie, par exemple par pulvérisation, atomisation et autres. L'hydrolysat protéique de l'invention peut être ajouté à un aliment pour animaux de compagnie par enrobage, habituellement en un mélange avec un ou plusieurs facteurs d'appétence et/ou graisse.

Le terme « **inclusion** » tel qu'il est utilisé ici désigne l'addition d'hydrolysat de l'invention dans le coeur de l'aliment pour animaux de compagnie. Par exemple, l'inclusion dudit hydrolysat dans un aliment pour animaux de compagnie peut être réalisée en le mélangeant avec d'autres ingrédients alimentaires pour animaux de compagnie, avant d'autres étapes de traitement, pour obtenir le produit alimentaire pour animaux de compagnie final (comprenant un traitement thermique et/ou une extrusion et/ou un autoclavage, etc.).

En particulier, l'aliment comprend entre 0.1% et 20%, de préférence entre 0.1% et 10% en poids dudit hydrolysat.

Selon ce mode de réalisation, l'hydrolysat protéique est présent dans un aliment en une quantité suffisante pour fournir une quantité de peptides (protéines solubles dans l'eau de poids moléculaire inférieur à 1000 Da) comprise entre 0.05 g et 10 g/100 g d'aliment, notamment entre 0.05 g et 5 g/100g d'aliment.

Ledit hydrolysat protéique est également présent dans la composition alimentaire de l'invention en une quantité suffisante pour fournir une quantité de phospholipides comprise entre 0.25 mg et 200 mg/100 g d'aliment, notamment entre 0.25 mg et 150 mg/100 g d'aliment.

Ledit hydrolysat protéique est aussi présent dans la composition alimentaire de l'invention en une quantité suffisante pour fournir une quantité de DHA et EPA comprise entre 0.5 mg et 150 mg/100 g d'aliment, notamment entre 0.5 mg et 100 mg/100 g d'aliment.

Ainsi, ledit hydrolysat protéique est présent dans la composition alimentaire de l'invention en une quantité suffisante pour permettre, sur la base d'une dose quotidienne, un apport journalier dudit hydrolysat allant de 0.02 grammes (gr) à 2 gr, de préférence de 0.02 gr à 1 gr par kilo de poids vif de l'animal.

Un autre aspect de la présente invention concerne un kit comprenant, dans un seul emballage, plusieurs contenants :
a) au moins un hydrolysat protéique tel que défini plus haut ;
b) au moins du DHA.

Selon un mode particulier de l'invention, ledit kit comprend en outre :
c) un ou plusieurs ingrédients alimentaires pour animaux de compagnie, choisis de préférence dans le groupe consistant en protéines, peptides, acides aminés, céréales, glucides, matières grasses ou lipides, nutriments, facteurs d'appétence, digestats animaux, farine de viande, gluten, conservateurs, tensioactifs, agents texturants ou agents de texture ou agents stabilisants, agents colorants, composés de phosphate inorganique, arômes et/ou assaisonnements.

Selon un autre mode de réalisation de l'invention, ledit kit comprend, dans un seul emballage, plusieurs contenants :
a) au moins un hydrolysat protéique tel que défini plus haut ;
b) un ou plusieurs ingrédients alimentaires pour animaux de compagnie, choisis de préférence dans le groupe consistant en protéines, peptides, acides aminés, céréales, glucides, matières grasses ou lipides, nutriments, facteurs d'appétence, digestats animaux, farine de viande, gluten, conservateurs, tensioactifs, agents texturants ou agents de texture ou agents stabilisants, agents colorants, composés de phosphate inorganique, arômes et/ou assaisonnements.
c) optionnellement au moins du DHA.

Des kits particuliers selon la présente invention comprennent en outre un moyen de communication d'informations ou d'instructions, pour aider à utiliser les éléments du kit.

Lesdits moyens de communication d'informations ou d'instructions sont donc un élément optionnel du kit.

Les « **contenants** » comprennent, de manière non restrictive, les sachets, les boîtes, les cartons, les bouteilles, les emballages de tout type ou de toute forme ou tout matériau, les sur-emballages, les emballages rétractables, les composants empilés ou fixés d'une autre façon ou leurs combinaisons, qui sont utilisés pour stocker des matériaux.

L'expression « **emballage unique** » ou « **un seul emballage** » signifie que les composants d'un kit sont physiquement associés dans ou avec un ou plusieurs contenants et sont considérés comme une unité pour la fabrication, la distribution, la vente ou l'utilisation. Un emballage unique peut consister en contenants de composants individuels physiquement associés, de sorte qu'ils sont considérés comme une unité pour la fabrication, la distribution, la vente ou l'utilisation.

Tel qu'elle est utilisée ici, l'expression « **moyen de communication d'informations ou d'instructions** » est un composant en kit sous une forme quelconque appropriée pour fournir des informations, des instructions, des recommandations, et/ou des garanties, etc. Ces moyens peuvent comprendre un document, un support de stockage numérique, un support de stockage optique, une présentation audio, un affichage visuel contenant des informations. Les moyens de communication peuvent être affichés sur un site internet, une brochure, une étiquette de produit, une notice d'emballage, une publicité, un affichage visuel, etc.

### Composition pharmaceutique

Selon un autre aspect, l'invention porte sur une composition pharmaceutique comprenant au moins un hydrolysat protéique selon l'invention, et tel que défini ci-dessus, un véhicule pharmaceutiquement acceptable et/ou un excipient.

La composition pharmaceutique de l'invention peut être administrée par voie systémique, par exemple, par voie orale, parentérale et dans certains cas même par voie transdermique. Chacune de ces formes d'administration sera plus ou moins adaptée en fonction de la situation réelle du sujet qui a besoin de ce traitement.

Par « **sujet** », on entend un mammifère, humain ou animal, de préférence un humain ou un animal de compagnie, de préférence encore un humain, un chien ou un chat, de sexe masculin ou féminin, de préférence âgé(e) et à risque de développer des troubles cognitifs liés au vieillissement.

Dans un mode de réalisation préféré, la composition pharmaceutique de l'invention est administrée par voie orale.

Dans un mode de réalisation préféré, la composition pharmaceutique de l'invention se présente sous la forme d'une poudre comprenant au moins un hydrolysat protéique selon l'invention.

Sous la forme d'une poudre, la composition pharmaceutique de l'invention peut être conditionnée sous forme de capsules, de gélules, de tablettes, de sachets, de comprimés, notamment de comprimés solubles ou effervescents en vue d'une administration orale.

Dans un mode de réalisation préféré, la composition pharmaceutique de l'invention est conditionnée sous forme de capsules ou de gélules à avaler.

Dans un autre mode de réalisation préféré, la composition pharmaceutique de l'invention est conditionnée dans des sachets sous forme de poudre à dissoudre.

La présente invention concerne encore des produits comprenant un hydrolysat protéique selon l'invention et du DHA comme produit de combinaison pour une utilisation simultanée, séparée ou étalée dans le temps comme médicament.

Un tel produit de combinaison peut, selon d'autres modes de réalisation, être utilisé notamment comme agent neuroprotecteur et/ou pour prévenir et/ou traiter la neuroinflammation et/ou dans le traitement ou la prévention des troubles cognitifs légers liés à l'âge et/ou prévenir et/ou limiter les troubles anxieux et/ou prévenir et/ou limiter le stress et/ou prévenir et/ou traiter les troubles de mémoire et/ou améliorer et/ou favoriser l'apprentissage spatial, de préférence liés au vieillissement.

Dans le cadre de la présente invention, le terme « **administration simultanée** » signifie que l'hydrolysat protéique et le DHA sont administrés ensemble au moyen d'une seule et même composition pharmaceutique et/ou alimentaire.

Dans le cadre de la présente invention, le terme « **administration séparée** » signifie que l'hydrolysat protéique et le DHA sont administrés en même temps au moyen de deux ou plus, compositions pharmaceutiques et/ou alimentaires distinctes.

Dans le cadre de la présente invention, le terme « **étalée dans le temps** » signifie que l'hydrolysat protéique et le DHA sont administrés de manière successive au moyen de deux ou plus compositions pharmaceutiques et/ou alimentaires distinctes.

Lorsque l'hydrolysat protéique et le DHA sont administrés de manière successive, leur administration peut intervenir dans un intervalle de temps pouvant aller de 0 à 120 minutes, notamment de 0 à 60 minutes, de préférence de 0 à 30 minutes et de préférence encore de 0 à 5 minutes.

### Utilisation thérapeutique

De manière surprenante, les inventeurs ont pu mettre en évidence que l'hydrolysat protéique selon l'invention (ou hydrolysat pur en poudre), tel que décrit ci-dessus, possède des vertus protectrices contre les troubles cognitifs légers liés à l'âge, notamment prévenir et/ou limiter les troubles anxieux, prévenir et/ou limiter le stress, prévenir et/ou traiter les troubles de mémoire, améliorer et/ou favoriser l'apprentissage spatial. Ainsi, les inventeurs ont pu montrer que l'hydrolysat protéique de l'invention avait en particulier un effet neuroprotecteur et un effet anti-neuroinflammatoire.

Ainsi, un autre objet de l'invention porte sur un hydrolysat protéique selon l'invention ou composition pharmaceutique en contenant, pour une utilisation comme médicament.

Une telle utilisation comme médicament peut être une utilisation comme agent neuroprotecteur et/ou une utilisation pour prévenir et/ou traiter la neuroinflammation et/ou une utilisation dans le traitement ou la prévention des troubles cognitifs légers liés à l'âge et/ou une utilisation pour prévenir et/ou limiter les troubles anxieux et/ou prévenir et/ou limiter le stress et/ou prévenir et/ou traiter les troubles de mémoire et/ou améliorer et/ou favoriser l'apprentissage spatial, de préférence lié au vieillissement.

L'invention porte encore sur un hydrolysat protéique selon l'invention ou composition pharmaceutique en contenant, pour une utilisation comme agent neuroprotecteur.

L'invention porte également sur un hydrolysat protéique selon l'invention ou composition pharmaceutique en contenant, pour une utilisation pour prévenir et/ou traiter la neuroinflammation.

L'invention porte également sur un hydrolysat protéique selon l'invention ou composition pharmaceutique en contenant, pour une utilisation dans le traitement ou la prévention des troubles cognitifs légers liés à l'âge.

L'invention porte également sur un hydrolysat protéique selon l'invention ou composition pharmaceutique en contenant, pour une utilisation pour prévenir et/ou traiter les troubles liés au vieillissement, prévenir et/ou limiter les troubles anxieux, prévenir et/ou limiter le stress, prévenir et/ou traiter les troubles de mémoire, améliorer et/ou favoriser l'apprentissage spatial.

Le terme « médicament » désigne ici autant un produit à destination des humains qu'un produit vétérinaire à destination des animaux de compagnie. Le médicament peut être administré de façons très différentes en fonction de son mode d'action et de sa capacité à être absorbé par le sujet auquel il est administré. Un médicament peut donc être administré, par exemple, par voie topique sous forme de formulations à appliquer ou « spot-on », sous forme de shampooings, de douches, sous forme de trempage, de bain ou de pulvérisation, sous forme de collier pour animaux et dans de nombreuses variantes de ces formes d'application. Il peut également être administré par voie systémique, par exemple, par voie orale, parentérale et dans certains cas même par voie transdermique. Chacune de ces formes d'administration sera plus ou moins adaptée en fonction de la situation réelle et de l'animal de compagnie ou de l'Homme qui a besoin de ce traitement.

Par « **traitement** », on entend le fait de diminuer les troubles cognitifs légers liés à l'âge. Une telle diminution peut être mise en évidence par le biais d'analyse montrant une diminution des troubles anxieux, et/ou du stress, et/ou des troubles de mémoire et/ou une amélioration l'apprentissage spatial. Avantageusement, le traitement permet d'éliminer complètement les troubles cognitifs légers liés à l'âge, mais le terme « traitement » recouvre toute diminution significative de ces troubles. Dans le cadre du traitement des troubles cognitifs légers liés à l'âge, la composition selon l'invention peut être associée à un autre traitement usuel des troubles cognitifs légers liés à l'âge bien connus de l'homme du métier.

Par « **prophylaxie** », on entend le fait de diminuer la probabilité de survenue de troubles cognitifs légers liés à l'âge. Cependant, le terme « prophylaxie » recouvre également la possibilité de diminuer significativement la fréquence de survenue des troubles cognitifs légers liés à l'âge dans une population de sujets ingérant une quantité efficace d'hydrolysat protéique selon l'invention pendant la durée de prise, par rapport à une population de sujets similaires ne prenant pas d'hydrolysat protéique selon l'invention (auquel cas la probabilité de troubles cognitifs légers liés à l'âge pendant la prise de l'hydrolysat protéique de l'invention est simplement diminuée significativement). Pour une telle comparaison, les populations comparées doivent être similaires, notamment concernant la proportion de sujets à risque de présenter des troubles cognitifs légers liés à l'âge.

Ainsi, l'administration orale des compositions de l'invention est particulièrement avantageuse dans le cas de la prophylaxie des troubles cognitifs légers liés à l'âge. En effet, l'administration régulière de l'hydrolysat de l'invention par voie orale permet de prévenir l'inflammation puis de maintenir sur le long terme un faible niveau inflammatoire dans les cellules microgliales et neuronales, notamment au niveau hippocampique.

L'invention porte encore sur l'utilisation d'un hydrolysat protéique, ou d'une composition pharmaceutique en contenant, pour la fabrication d'un médicament destiné au traitement ou à la prophylaxie des troubles cognitifs légers liés à l'âge.

De façon avantageuse, ledit hydrolysat protéique est tel que décrit ci-dessus.

L'hydrolysat de l'invention peut être utilisée dans une méthode de traitement prophylactique ou thérapeutique des troubles cognitifs légers liés à l'âge comprenant l'administration à un sujet en ayant besoin d'une quantité efficace d'un hydrolysat protéique, ou d'une composition pharmaceutique en contenant.

L'hydrolysat de l'invention peut être utilisé dans une méthode de traitement prophylactique ou thérapeutique des troubles cognitifs légers liés à l'âge comprenant l'administration simultanée, séparée ou étalée dans le temps à un sujet en ayant besoin d'une quantité efficace de produits contenant un hydrolysat protéique et du DHA, comme produit de combinaison.

Dans le cadre de la présente méthode de traitement, ledit hydrolysat protéique est tel que décrit ci-dessus.

Une « **quantité efficace** » d'un hydrolysat protéique ou d'une composition pharmaceutique en contenant, telle qu'utilisée ici est une quantité de l'hydrolysat protéique ou d'une composition pharmaceutique en contenant fourni par une voie d'administration particulière et selon un mode d'administration particulier, qui est suffisante pour obtenir un effet thérapeutique et/ou prophylactique souhaité tel que défini ci-dessus. La quantité d'hydrolysat protéique ou d'une composition pharmaceutique en contenant administrée au sujet dépendra du type et de la sévérité de la maladie, du type, de l'âge, du poids corporel, de l'état de santé général, du sexe et du régime alimentaire du sujet ; du moment d'administration, de la voie d'administration et de la vitesse d'élimination du composé spécifique employé ; de la durée du traitement ; des médicaments utilisés en combinaison ou de façon concomitante avec l'hydrolysat ou la composition pharmaceutique en contenant ; et facteurs similaires bien connus dans l'art médical. L'homme du métier saura déterminer les dosages appropriés en fonction de ces facteurs ainsi que d'autres. Par exemple, l'homme du métier sait bien utiliser pour commencer des doses du composé à des niveaux inférieurs à ceux requis pour obtenir l'effet thérapeutique et/ou prophylactique souhaité et augmenter progressivement le dosage jusqu'à ce que l'effet souhaité soit obtenu.

La présente invention sera décrite en détail en référence aux exemples suivants, qui sont présentés à des fins d'illustration uniquement et ne sont pas destinés à limiter le cadre de l'invention.

### EXEMPLES

Les hydrolysats ci-dessous illustrés conformément à la présente invention sont des hydrolysats purs en poudre tels que décrits plus haut.

### Exemple 1 : Matériel et Méthodes

### 1.1. Cultures cellulaires

### BV2 :

Les cellules BV2 sont issues d'une lignée de cellules microgliales murines néonatales immortalisées par le système raf/mycet, fournies par Dr. Watterson (Université NorthWestern, USA). Elles ont été cultivées dans un milieu complet contenant du RPMI-Glutamax avec 2 mM de glutamine (Invitrogen, Life Technologies, France) additionné de 10% de sérum de veau foetal inactivé et de 1% d'un mélange de pénicilline (100 U/mL)-streptomycine (100 µg/mL ; Sigma-Aldrich, France) sous une atmosphère humide à 5% de CO2 à 37°C comme décrit par De Smedt-Peyrusse et al. (8).

Lorsque les cellules ont atteint 80% à 90% de confluence, celles-ci sont traitées pendant 24h avec :
- du DHA à la concentration de 16µM (Sigma-Aldrich, France). Cette concentration en DHA a été validée au laboratoire comme étant une dose efficace pour mettre en évidence un effet anti-inflammatoire.
- de l'hydrolysat selon l'invention apportant 16µM de DHA.

Les cellules ont ensuite été traitées pendant 2h, 6h ou 24h avec 1 µg/mL de LPS (lipopolysaccharides) (Sigma-Aldrich) afin d'induire un stress inflammatoire puis collectées dans du Trizol (n=6 ; Invitrogen, Life Technologies).

### Coculture :

Afin d'avoir un modèle plus complexe, une coculture dite en « sandwich » a été réalisée entre des cellules neuronales (HT22) et des cellules microgliales (BV2). Dans un premier temps, les 2 types de cellules sont mis en culture séparément. Les cellules HT22 sont issues d'une lignée de cellules hippocampiques de souris, fournies par le Dr. E. Maronde (Allemagne). Elles ont été cultivées dans un milieu complet contenant du DMEM (Invitrogen, Life Technologies) additionné de 10% de sérum de veau foetal inactivé et de 1% d'un mélange de pénicilline (100 U/mL)-streptomycine (100 µg/mL ; Sigma-Aldrich) sous une atmosphère humide à 5% de CO2 à 37°C.

Les BV2 sont mises en culture dans du milieu complet RPMI dans des plaques 6 puits simples et les HT22 dans du milieu complet DMEM sur des lamelles. Après 24h de culture, les HT22 sont retournées sur le tapis microglial afin de traiter l'ensemble des deux lignées cellulaires pendant 16h avec l'hydrolysat selon l'invention apportant 16µM de DHA.

Les cellules ont ensuite été traitées pendant 6h avec 1 µg/mL de LPS pour induire un stress inflammatoire puis collectées séparément (n=5).

### 1.2. Animaux et Traitements

Toutes les expériences ont été menées sur des souris mâles C57Bl/6J (Janvier) âgées de 7 semaines et de 12 mois.

Les souris ont été placées en cage individuelle afin de contrôler leur prise alimentaire et leur prise de poids. Pendant 12 semaines, elles ont reçu *ad libitum* un régime déficient en AGPI n-3 permettant de mimer les déficits en AGPI n-3 liés à l'âge. A l'issue de cette période, les animaux reçoivent une supplémentation en hydrolysat.

Pour l'hydrolysat H1, les animaux ont été répartis en 4 groupes supplémentés ou non :
▪ Jeune Témoin (n=12), recevant le régime déficient en AGPI n-3.
▪ Âgé Témoin (n=12), recevant le régime déficient en AGPI n-3.
▪ Jeune H1 (n=12), recevant l'hydrolysat H1 selon l'invention.
▪ Âgé H1 (n=12), recevant l'hydrolysat H1 selon l'invention.

**Tableau 1 : hydrolysat H1**

| [Table 1] | | | |
|---|---|---|---|
| g/kg de régime | | Régime Témoin | Régime H1 |
| Hydrolysat H1 selon l'invention | | - | 200 |
| | *Peptides* | - | *140* |
| | *DHA* | - | *3.05* |

Pour l'hydrolysat H2, les animaux ont été répartis en 6 groupes supplémentés ou non :
▪ Jeune Témoin (n=12), recevant le régime déficient en AGPI n-3.
▪ Âgé Témoin (n=13), recevant le régime déficient en AGPI n-3.
▪ Jeune H2 (n=12), recevant l'hydrolysat H2 selon l'invention.
▪ Âgé H2 (n=13), recevant l'hydrolysat H2 selon l'invention.
▪ Jeune H2 + DHA (n=12), recevant l'hydrolysat H2 selon l'invention enrichi en DHA.
▪ Âgé H2 + DHA (n=13), recevant l'hydrolysat H2 selon l'invention enrichi en DHA.

**Tableau 2 : hydrolysat H2**

| [Table 2] | | | | |
|---|---|---|---|---|
| g/kg de régime | | Régime Témoin | Régime H2 | Régime H2+DHA |
| Hydrolysat H2 selon l'invention | | - | 1.66 | 1.66 |
| | *Peptides* | - | *1.11* | *1.11* |
| | *DHA* | - | *0.029* | *0.0352* |
| DHA (Polaris) | | - | - | 2 |

Après 6 semaines de supplémentation, la totalité des souris a été soumise à des tests comportementaux évaluant leurs capacités cognitives et leur réactivité au stress. A l'issue de ces protocoles, les animaux ont été euthanasiés et les structures d'intérêt, dont l'hippocampe, ont été prélevées. L'effet des supplémentations sur l'expression des facteurs inflammatoires et neurotrophiques a été déterminé par PCR quantitative en temps réel. L'étude de l'inflammation aiguë a été menée sur des souris mâles C57Bl/6J (Janvier) âgées de 7 semaines.

Les souris ont été placées par 6 dans des cages collectives, avec accès *ad libitum* à l'eau et à un régime standard (régime A04, Safe, Augy, France) puis ont été gavées pendant 18 jours via une sonde gastrique (Ecimed, Boissy-Saint-Léger, France). Les souris ont été réparties en 3 groupes supplémentés ou non :
▪ Témoin (n=11), recevant 100µL d'eau + 50µL d'huile d'arachide
▪ H2 (n=10), recevant 100µL de l'hydrolysat H2 + 50µL d'huile d'arachide
▪ DHA (n=12), recevant 100µL d'eau + 50µL de DHA (Polaris, Quimper, France)

**Tableau 3 : Hydrolysat H2 gavage**

| | | Témoin | H2 | DHA |
|---|---|---|---|---|
| Eau (µL/jour) | | 100 | - | 100 |
| Huile d'arachide (µL/jour) | | 50 | 50 | - |
| Hydrolysat H2 selon l'invention (µL/jour) | | - | 100 | - |
| | *Peptides (mg*/*jour)* | *-* | *5.5* | - |
| | *DHA (mg*/*jour)* | - | *0.143* | - |
| DHA (µL/jour)(10mg/jour *) | | - | - | 50 |

| | | | | |
|---|---|---|---|---|
| * : dose efficace prouvée pour un effet cognition | | | | |

A l'issue des 18 jours de supplémentation, les souris ont reçu une injection intrapéritonéale de 125µg/kg de LPS (Escherichia coli, 0127 : B8, Sigma-Aldrich, Lyon, France) (Rey et al., 2019 (9) ; Mingam et al., 2008 (10)) afin d'induire une réaction inflammatoire ou bien une injection de solution saline (NaCl 0,9%). Deux heures après l'injection, les souris ont été euthanasiées et les structures d'intérêt, dont l'hippocampe, ont été prélevées.

### 1.3. Tests comportementaux

### 1.3.1. Labyrinthe en Y :

6 semaines après le début des supplémentations, la mémoire de reconnaissance spatiale, dépendante de l'hippocampe, a été évaluée grâce au labyrinthe en Y comme décrit par Labrousse et al. (11) et Moranis et al. (12). Ce test de mémoire spatiale hippocampo-dépendante est basé sur la curiosité des rongeurs et sur leur capacité à discerner un environnement nouveau d'un environnement familier. Il permet ainsi une évaluation de la mémoire spatiale liée à l'hippocampe. Ce test est réalisé dans un labyrinthe en forme de Y comprenant 3 bras (35 cm de long et 10 cm de profondeur), éclairé à 15 lux. Des repères visuels sont disposés sur les murs, permettant aux souris de se repérer dans l'espace.

Dans un premier temps, l'animal est placé dans un bras de départ et fait face aux 2 autres bras dont l'un est fermé. Il dispose de 5 minutes pour explorer les 2 bras ouverts puis est replacé dans sa cage pendant 1 heure. Au cours de la phase de restitution, l'animal est de nouveau placé dans le labyrinthe durant 5 minutes, mais cette fois-ci avec les 3 bras ouverts. De par leur comportement exploratoire fort, les animaux vont préférentiellement explorer le bras nouveau. Les animaux explorant de façon aléatoire les 3 bras ont des capacités mnésiques altérées.

Les animaux sont filmés et vidéotrackés (logiciel SMART, Bioseb) afin d'analyser le temps passé (minutes) dans les différents bras. De plus, un indice de reconnaissance a été calculé afin de comparer les performances des animaux par rapport au hasard (33%) : temps passé dans le nouveau bras/ (temps passé dans le nouveau bras + temps passé dans le bras familier + temps passé dans le bras de départ).

### 1.3.2. « Open-Field » :

7 semaines après le début des supplémentations, le comportement de type anxieux des animaux a été évalué grâce au test de l' « Open-Field » (champ ouvert). Ce test est basé sur l'aversion que produit un environnement inconnu et ouvert. Le test est effectué grâce à un dispositif opaque (25 cm de large, 45 cm de long et 40 cm de profondeur) éclairé à 30 lux. Le dispositif est délimité virtuellement en deux parties : la partie centrale considérée comme anxiogène, et la partie périphérique considérée comme étant moins anxiogène. Les animaux sont libres d'explorer l'open-field pendant 10 minutes, ils sont filmés et vidéotrackés (logiciel SMART, Bioseb) afin de mesurer le temps passé (minutes) dans les différentes zones.

### 1.3.3. Labyrinthe aquatique de Morris :

7 semaines après le début des supplémentations, les capacités d'apprentissage et de mémoire spatiale ont été évaluées grâce au labyrinthe aquatique de Morris (13). Le protocole expérimental utilisé pour cette étude met plus particulièrement en jeu la mémoire spatiale de référence dépendante de l'hippocampe.

Le protocole utilisé est celui décrit par Bensalem *et al.* (14). Il se compose de 4 phases :
▪ La *familiarisation* : permet aux souris de se familiariser à la nage et au fait de monter sur une plateforme dans une bassine (60 cm de diamètre), pendant 2 jours (jours 1 et 2).
▪ *L'apprentissage indicé* : réalisé au 3^{ème} jour, il permet d'évaluer les déficits moteurs et visuels et d'accentuer la familiarisation. Les animaux doivent trouver une plateforme immergée indicée.
▪ *L'apprentissage spatial de référence* : les animaux doivent apprendre à retrouver la plateforme immergée non visible à l'aide d'indices visuels spatiaux (jours 4 à 7). Pour cela, les souris ont 6 essais/jour (cut-off de 90s) pendant 4 jours consécutifs. La vitesse de nage, la latence pour atteindre la plateforme, la distance et le chemin parcourus par les animaux pour chaque essai ont été enregistrés par un système de vidéotracking (Imetronic).

### 1.3.4. Le test de rappel « probe test » :

Ce test permet d'évaluer la mémoire spatiale. 72h après le dernier jour d'apprentissage, la plateforme est retirée de la piscine et l'animal est placé dans la piscine pendant 60 secondes dans la piscine. Les animaux sont vidéotrackés (logiciel SMART, Bioseb) afin de mesurer le temps passé (secondes) et la distance parcourue (cm) dans le quadrant « cible », c'est-à-dire le quadrant où était localisée la plateforme lors de la phase d'apprentissage spatial.

### 1.3.5. Analyse des stratégies d'apprentissage :

Pour chaque essai au cours de l'apprentissage spatial, les trajectoires de nage ont été analysées grâce au replay du logiciel de vidéotracking Imetronic. Les stratégies de recherche de la plateforme ont été classifiées à l'aveugle puis assignées pour chaque essai en utilisant un patron de classification similaire à ceux précédemment développés pour d'autres études (15, 16, 17). Ces stratégies ont été divisées en deux catégories principales : stratégies non-spatiales et stratégies spatiales.

Les stratégies non-spatiales se composent d'abord des stratégies de « recherche globale » : la « recherche périphérique » (l'animal nage préférentiellement dans une zone de 15 cm autour des bords de la piscine), « au hasard » (recherche dans toute la piscine en couvrant >75% de la surface), « en cercle » (la souris fait de tout petits cercles et peut faire quelques mouvements nets dans certaines directions). On retrouve ensuite des stratégies de « recherche locale » : le « balayage » (l'animal cherche dans une zone limitée, souvent au centre, de la piscine en couvrant en 15 et 75% de la surface), la « boucle » (la souris nage de façon circulaire à une distance assez fixe des bords supérieure à 15cm), la « incorrecte répétée » (l'animal nage dans une direction précise qui ne correspond pas à la position de la plateforme et répète la même trajectoire à plusieurs reprises), et enfin la « focale incorrecte » (l'animal cherche de façon intensive dans une petite portion de la piscine qui ne contient pas la plateforme).

Les stratégies spatiales comportent les stratégies « correcte répétée » (lorsque l'animal nage dans la direction de la plateforme puis répète la même trajectoire à plusieurs reprises), « spatiale indirecte » (l'animal nage indirectement vers la plateforme en effectuant éventuellement une ou deux boucles), « spatiale directe » (la souris nage directement vers la plateforme), et la « focale correcte » (l'animal nage puis cherche de façon intensive dans le quadrant où est située la plateforme).

### 1.3.6. Réactivité au stress :

La réactivité au stress a été effectuée 10 semaines après le début des supplémentations. Pour ce faire les animaux sont soumis à un test de contention de 30 minutes. Un prélèvement de sang à la joue est effectué au niveau de la veine mandibulaire au début du test (T0), puis 30 minutes (T30) et 60 minutes (T60) après le début du test. Les souris sont euthanasiées 90 minutes (T90) après le test et une ponction intracardiaque est réalisée afin de prélever un échantillon de sang.

### 1.4. Analyses biochimiques

### 1.4.1. Dosage des taux de corticostérone plasmatique :

Le plasma a été isolé du sang par centrifugation à 3000g pendant 20 minutes. A partir du plasma, un dosage de la corticostérone totale a été réalisé grâce au Kit ELISA DetectX^{®} « Corticostérone, Enzyme Immunoassay Kit » selon les indications du fournisseur (Arbor Assay). La concentration en corticostérone (ng/mL) de chaque échantillon est calculée en fonction de la gamme étalon obtenue par spectrophotométrie.

### 1.4.2. Expression génique :

L'expression des différents gènes d'intérêt a été évaluée par PCR quantitative en temps réel comme décrit par Rey *et al.* (18). Ces analyses ont été réalisées sur les cellules BV2 et HT22 et sur les hippocampes.

### 1.4.3. Extraction des ARN totaux :

Les ARN totaux ont été extraits des cellules et des hippocampes en utilisant le protocole d'extraction du réactif TRIzol (Invitrogen, Life Technologies) contenant du phénol et de l'isothiocyanate de guanidine. Ce réactif permet, après lyse et ajout de chloroforme, de séparer une phase aqueuse supérieure contenant l'ARN d'une phase organique contenant l'ADN et les protéines. Après récupération de la phase aqueuse, l'ARN est précipité avec du glycogène (20mg/mL) et de l'isopropanol. Après des lavages successifs à l'éthanol 70%, le culot d'ARN est séché puis repris dans 10µL d'eau stérile. La pureté et la quantité d'ARN de chaque échantillon ont été mesurées par spectrophotométrie (Nanodrop, Life technologies).

### 1.4.4. Transcription inverse :

La transcription inverse (RT) a été réalisée avec 1µg ou 2µg d'ARN, selon la quantité obtenue, pour synthétiser des ADN complémentaires (ADNc). Les ARN ont été incubés à 37°C pendant 15 minutes puis à 75°C durant 1 minute avec un mélange de tampon DNAse, RNAsin, DNAse I (Invitrogen, Life Technologies). Une deuxième incubation à 65°C pendant 5 minutes en présence de random primers (150ng/µL, Invitrogen) et de dNTP à 10mM est effectuée. Enfin, un mélange de tampon 5X, de DTT, de RNase OUT à 40U/µL et de Supercript III à 200U/µL (Invitrogen, Life Technologies) a été ajouté. Le mix réactionnel est incubé à 25°C pendant 5 minutes, puis 50°C pendant 50 minutes et 70°C pendant 15 minutes. La concentration finale des ADNc obtenus est de 50ng/µL (si 1µg d'ARN initial) ou 100ng/µL (si 2µg d'ARN initial).

### 1.4.5. PCR quantitative en temps réel :

Les ADNc issus de la réaction de RT ont été amplifiés sélectivement grâce à des amorces spécifiques aux séquences des gènes cibles étudiés. Le gène de référence utilisé ici est la β-Actine, et les gènes cibles étudiés, pour la coculture, sont : IL-6, IL-1β, TNF-α, BDNF et NGF. Pour l'étude de l'inflammation chronique, les gènes cibles suivants ont été amplifiés : dans l'hippocampe IL-6, IL-1β, TNF-α, CD11b et Iba1, et dans l'hypothalamus : CrhR1, CRHBP, HSD11b1. Pour l'étude de l'inflammation aigüe, les gènes cibles suivants ont été amplifiés pour les hippocampes : IL-6, IL-1β, TNF-α, COX-2, BDNF et NGF.

Pour chaque échantillon, 2µl d'ADNc à 20ng/pl ont été additionnés à 8µl d'un mélange comprenant de la Taq polymérase (5X), les couples d'oligonucléotides des gènes cibles (2X) et de l'eau stérile (1X). La plaque a ensuite été placée dans un thermocycler Light Cycler (LC 480 version 2, Roche) afin de réaliser le programme de PCR : une phase d'activation (2 minutes à 95°C) et 50 cycles d'amplification comprenant chacun une phase de dénaturation (15 secondes à 95°C), et une phase d'hybridation des oligonucléotides et élongation (1 minute à 60°C).

La quantification finale a été réalisée en utilisant la méthode comparative des « Cycle Threshold » (Ct). Pour chaque gène cible et chaque échantillon, le taux de transcrits a été normalisé avec le taux de transcrits du gène de référence (β-Actine).

### 1.4.6. Carte Biomark

Les ADNc issus de la RT ont été dilués (1.3 µL, 5 ng/µL) puis ont été ajoutés au DNA Binding Dye Sample Loading Reagent (Fluidigm), à l'EvaGreen (Interchim, Montluçon, France) et au tampon Tris-EDTA (TE) à faible EDTA pour constituer la plaque d'échantillons. Dans la plaque de Mix, 10µL de paires d'amorces (100µM) ont été ajoutés au réactif de chargement d'essai (Fluidigm) et au tampon TE à faible EDTA pour obtenir une concentration finale de 5µM. Après l'activation de la puce dans le contrôleur de circuit fluidique intégré, le mélange d'échantillons (5µL) et le mélange d'essai (5µL) ont été chargés dans les puits d'entrée des échantillons. Un des puits a été rempli d'eau pour contrôler la contamination. Pour vérifier l'amplification de cibles spécifiques et l'efficacité du processus de réaction en chaîne de la polymérase quantitative (qPCR), un échantillon témoin (ADNg de souris, ThermoFisher, Waltham, USA) a été traité, préamplifié et quantifié dans un essai témoin (sonde RNasePTaqMan, ThermoFisher) en utilisant le même processus dans la même plaque au même moment. La puce a été insérée dans le contrôleur IFC, dans lequel 6,3nL de mélange d'échantillons et 0,7nL de Mix ont été mélangés. La PCR en temps réel a été réalisée à l'aide du système de biomarqueurs (Fluidigm) sur la plateforme GenoToul (Toulouse, France) avec le protocole suivant : Mélange thermique à 50°C, 2 min ; 70°C, 30 min ; 25°C, 10 min, Uracil-ADN N-glycosylase (UNG) à 50°C, 2 min, démarrage à chaud à 95°C, 10 min, cycle de PCR de 35 cycles à 95°C, 15s ; 60°C, 60 s et courbes de fusion (de 60°C à 95°C). Les résultats ont été analysés à l'aide du logiciel d'analyse PCR en temps réel Fluidigm v.4.1.3. (San Francisco, États-Unis) pour contrôler l'amplification spécifique de chaque amorce. Ensuite, les données brutes de la qPCR ont été analysées à l'aide du logiciel GenEx (MultiD analyses AB, Freising, Allemagne) afin de choisir le meilleur gène de référence, ici la β-Actine, pour la normalisation de l'expression de l'ARNm et de mesurer l'expression relative de chacun des 46 gènes analysés.

### 1.4.7. Expression protéique

Le Western blot permet la détection de protéines cibles à l'aide d'anticorps dirigés contre ces protéines. Les échantillons ont été dilués avec de l'eau RNase-Free afin d'obtenir une concentration égale à 1µg/µL dans 200µL. Un volume de 50µL de chaque échantillon a été prélevé auquel il a été ajouté 12,5µL de tampon de charge. Les échantillons ont ensuite été chauffés à 75°C pendant 5 minutes afin de dénaturer les protéines.

L'électrophorèse SDS-PAGE en conditions dénaturantes permet la migration des protéines dans un champ électrique uniquement en fonction de leur poids moléculaire. Le gel de polyacrylamide a été coulé puis recouvert d'un tampon de migration qui permet la migration des protéines. Les échantillons, ainsi qu'un marqueur de taille, ont été déposés dans les puits du gel puis ont migré à 90V pendant 30 minutes et 130V pendant 1 heure. Afin de pouvoir détecter les protéines, elles ont été transférées sur membrane de nitrocellulose (75V pendant 1h30 avec du tampon de transfert). Pour vérifier le bon fonctionnement du transfert, les membranes ont été colorées au rouge Ponceau puis rincées. Les sites non spécifiques ont été bloqués par une incubation dans une solution de TBS/Tween-20 0,05% (TBST) et lait 5% (lait écrémé Régilait) pendant 1 heure afin d'éviter les interactions entre la membrane et l'anticorps. Après rinçage au TBST, les membranes ont été incubées une nuit à 4°C dans une solution de BSA 5%, sodium azide 1% avec les anticorps primaires suivants : anti-GAPDH, anti-IκB. Après trois lavages successifs au TBST, les membranes ont été incubées 1 heure avec une solution d'anticorps secondaire de lapin conjugué à la peroxydase. Après cinq autres lavages au TBST et au TBS, les membranes ont été incubées 5 minutes dans une solution révélatrice de peroxydase (SuperSignal West Dura, ThermoFisher, Waltham, Etats-Unis). Les protéines ont ensuite été révélées à l'aide de l'appareil ChemiDoc MP (Biorad, Hercules, Etats- -Unis). Le ratio de l'intensité des bandes de la protéine cible sur celle des bandes de la protéine de référence (contrôle endogène, GAPDH) a été utilisé afin de comparer l'expression relative des protéines.

### 1.4.8 Analyse des acides gras cérébraux

Les lipides contenus dans le cortex ont été extraits (Folch et al., 1997 (19)) et les acides gras ont été transméthylés selon la méthode de Morrison et Smith (Morrison and Smith, 1964). Les esters méthyliques d'acides gras ont été analysés par chromatographie en phase gazeuse sur un modèle Hewlett Packard 5890 série II (Palo Alto, CA, USA) équipé d'un injecteur, d'un détecteur à ionisation de flamme (Palo Alto, CA, USA), et d'une colonne CPSIL-88 (100m×0,25mm de diamètre interne ; épaisseur du film, 0,20µm ; Varian, Les Ulis, France). Le gaz vecteur était de l'hydrogène (pression d'entrée, 210 kPa). La température du four a été maintenue à 60°C pendant 5 min, puis augmentée à 165°C à 15°C/min et maintenue pendant 1 min, puis à 225°C à 2°C/min et finalement maintenue à 225°C pendant 17 min. L'injecteur et le détecteur ont été maintenus à 250°C et 280°C, respectivement. Les esters méthyliques d'acides gras ont été identifiés par comparaison avec les standards. La composition en acides gras est exprimée en pourcentage du total des acides gras.

### 1.4.9. Quantification des oxylipines

Les différents métabolites dérivés de l'acide arachidonique (AA), l'acide linoléique (AL), du DHA et de l'EPA ont été extraits de l'hippocampe et analysés par spectrométrie de masse (LC-MS/MS) à la plateforme METATOUL (MetaboHUB, INSERM UMR 1048, I2MC, Toulouse, France) comme précédemment décrit par Le Faouder et al. 2013 (20).

### 1.5. Statistiques

Les analyses statistiques ont été réalisées avec les logiciels GraphPad Prism et Statistica. En ce qui concerne les analyses de l'Open Field, de la réactivité au stress et des qPCR, les 6 groupes expérimentaux ont été comparés par des ANOVA à 2 facteurs (âge et régime) suivi d'un test post-hoc de Tukey en cas d'interaction statistiquement significative entre les facteurs. Pour le labyrinthe en Y les 6 groupes expérimentaux ont été comparés par un one sample t-test par rapport à la valeur du hasard 33%. Pour l'analyse du labyrinthe aquatique de Morris, la phase d'apprentissage des 6 groupes expérimentaux a été analysée par une ANOVA à 3 facteurs à mesures répétées (âge, régime et jour) ; et le probe test a été analysé par une ANOVA 1 facteur (quadrants).

Les données ont été exprimées sous la forme de moyennes ± écart-type par rapport à la moyenne (SEM), et les différences ont été considérées comme significatives lorsque la valeur de p était inférieure à 0,05.

### 1.6. Préparation des hydrolysats

Un hydrolysat protéique, H1, a été obtenu par le procédé suivant : une solution aqueuse contenant 50% de broyat de têtes de sardines a été hydrolysée à 55°C par l'alcalase 2.4L, pendant une durée de 2 heures. L'enzyme a ensuite été inactivée par chauffage à 95°C pendant 30 minutes. Les particules solides (arêtes) ont été enlevées par tamisage. Une étape de centrifugation verticale a alors été réalisée, de manière à retirer les boues et la phase grasse (principalement des protéines insolubles, des lipides neutres - triglycérides -, de la matière minérale). La phase aqueuse a ainsi été isolée puis séchée.

Un hydrolysat protéique, H2, a été obtenu par le procédé suivant : une solution aqueuse contenant 87% de broyat de têtes de sardines a été hydrolysée à 55°C par l'alcalase 2.4L, pendant une durée de 3 heures. L'enzyme a ensuite été inactivée par chauffage à 95°C pendant 30 minutes. Les particules solides (arêtes) ont été enlevées par tamisage. Une étape de centrifugation verticale a alors été réalisée, de manière à retirer les boues et la phase grasse (principalement des protéines insolubles, des lipides neutres - triglycérides -, de la matière minérale). La phase aqueuse a ainsi été isolée puis séchée.

### 1.7. Caractérisation des hydrolysats

Plusieurs caractéristiques des hydrolysats H1 et H2 ont été déterminées, comme montré dans le tableau 4.

Le DH a été déterminé par (1) la méthode pH-stat, comme décrit par J. Adler-Nissen (21), ainsi que (2) par la méthode OPA, comme décrit par Nielsen, P (22).

L'humidité a été mesurée selon la méthode du Règlement CE 152/2009.

La quantité de protéines totales a été déterminée par la méthode Dumas, basée sur la norme NF EN ISO 16634-1.

La quantité de protéines solubles a été déterminée en solubilisant l'échantillon dans de l'eau ultrapure, en récupérant la phase aqueuse après centrifugation et en appliquant la méthode de Kjedahl (Règlement CE 152/2009) sur ladite phase aqueuse.

La quantité de matière minérale a été déterminée selon la méthode de la norme NF V04-404 - Avril 2001.

La quantité de matière grasse totale (ou lipides totaux) a été déterminée selon la méthode du Règlement CE 152/2009.

Les quantités de lipides neutres, dont les triglycérides, de lipides polaires, dont la phosphatidylcholine, ont été déterminées par HPTLC (High-Performance Thin-Layer Chromatography). Les différentes classes de lipides neutres et de lipides polaires ont été analysées séparément par HPTLC (High performance Thin layer chromatography) sur des plaques de verre (10x20 cm) imprégnées de silice 60 (Merck). Un lavage préliminaire de la plaque a été réalisé avec un mélange hexane / diéthyl éther (97:3, v/v) dans le cas des lipides neutres, et avec un mélange méthyl acétate / isopropanol / chloroforme / méthanol / KCl (0.25%) (10:10:10:4:3.6; v:v) dans le cas des lipides polaires, afin d'enlever les possibles impuretés. Les plaques ont ensuite été activées à 110°C pendant 30 minutes. Les extraits lipidiques ont été déposés en quantité adaptée suivant les échantillons par un passeur automatique (CAMAG). Un double développement a permis de séparer les lipides neutres. Lors de la première migration un mélange hexane / diéthyl éther / acide acétique (20/5/0.5, v:v:v) a été utilisé et un mélange hexane / diéthyl éther (93/3, v:v) lors de la deuxième. Les lipides polaires ont été séparés après un seul développement, avec comme solvant d'élution un mélange méthyl acétate / isopropanol / chloroforme / méthanol / KCl (0.25%) (10:10:10:4:3.6; v:v).

Après révélation par immersion des plaques dans une solution d'acide cuprique et d'acide phosphorique suivie d'un chauffage à 120°C pendant 20 minutes, les différentes classes de lipides neutres et de lipides polaires sont apparues sous forme de taches noires. Six classes de lipides neutres (alcools, acides gras libres, esters de stérols, éthers de glycérides, triacylglycérols et stérols) et 7 classes de lipides polaires (phosphatidyl ethanolamine, phosphatidyl choline, phosphatidyl sérine, phosphatidyl inositol, cardiolipide, céramide aminoéthylphosphonate et lysophosphatidyl choline) ont pu être séparées et identifiées par comparaison avec des standards. La quantification a été réalisée par étalonnage externe, par densitométrie à l'aide d'un scanner réglé à 370 nm et du logiciel Wincats (CAMAG). Les quantités d'oméga 3 et d'oméga 6, de DHA et EPA, de plasmalogènes ont été déterminées comme décrit par Mathieu-Resuge, et al., (23).

**Tableau 4 : Caractéristiques des hydrolysats H1 et H2**

| [Table 4] | | | |
|---|---|---|---|
| | | H1 | H2 |
| DH (pH-stat) (%) | | 16.0 | 12.8 |
| DH (OPA) (%) | | 32.2 | 30.3 |
| Humidité (g/100g) | | 2.6 | 3.4 |
| Protéines totales (g/100g) | | 72.1 | 69.6 |
| Protéines solubles (g/100g) | | 70.4 | 67.0 |
| Matière minérale (g/100g) | | 9.5 | 9.0 |
| Matière grasse totale (= lipides totaux) (g/100g) | | 7.8 | 10.3 |
| Phosphatidylcholine (mg/g) | | 10.0 | 8.9 |
| Oméga 3 (mg/g) | | 26.5 | 33.8 |
| Oméga 6 (mg/g) | | 3.6 | 4.5 |
| DHA (mg/g) | | 13.5 | 17.3 |
| EPA (mg/g) | | 6.6 | 8.4 |
| Plasmalogènes (mg/g) | | 0.2 | 0.3 |
| Lipides neutres (mg/g) | | 61.6 | 85.5 |
| | *Triglycérides* | 58 | *73.6* |
| Lipides polaires (mg/g) | | 16.4 | 18.2 |
| | *EPA* | *0.9* | *0.9* |
| | *DHA* | 4.9 | 4.4 |

Le profil de poids moléculaire des protéines solubles dans l'eau a été déterminé par chromatographie SEC comme décrit dans F. Guérard et al., (24).

**Tableau 5 - Profil de poids moléculaire des protéines solubles dans l'eau**

| [Table 5] | | |
|---|---|---|
| | H1 | H2 |
| > 5000 Da | 0.4 | 0.6 |
| 1000-5000 Da | 10.7 | 12.8 |
| 500-1000 Da | 14.4 | 12.9 |
| < 500 Da | 74.5 | 73.7 |

### Exemple 2 : Effet in vitro de l'hydrolysat

### 2.1. Effet de l'hydrolysat H1 sur la neuroinflammation des cellules microgliales BV2

Les cellules microgliales sont les cellules immunocompétentes du cerveau, responsables de la production des cytokines. Un stress inflammatoire a été induit dans ces cellules (cellules BV2) par le liposaccharide (LPS) selon le protocole décrit à l'Exemple 1.1. L'hydrolysat protéique H1 a alors été testé pour sa capacité à diminuer l'expression des cytokines pro-inflammatoires IL-6 (interleukine 6), IL-1β (interleukine 1beta) et TNF-α (tumor necrosis factor alpha), dans ces cellules, selon le protocole décrit à l'exemple 1.4.5.

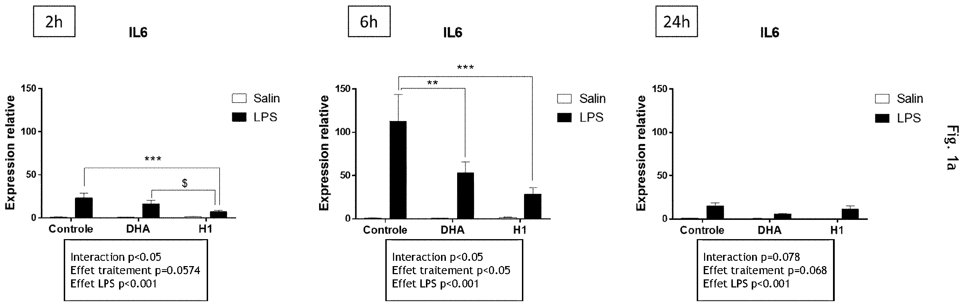

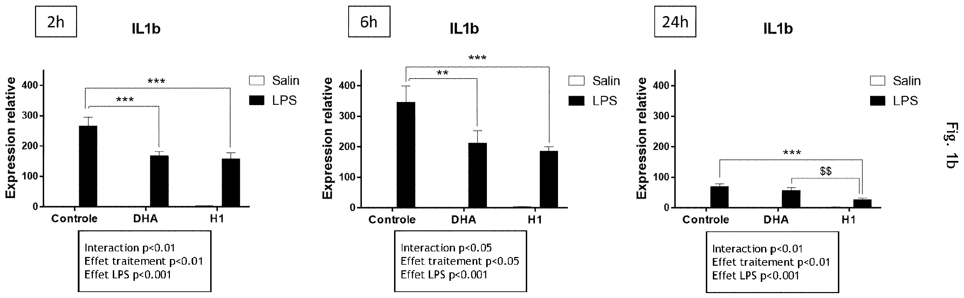

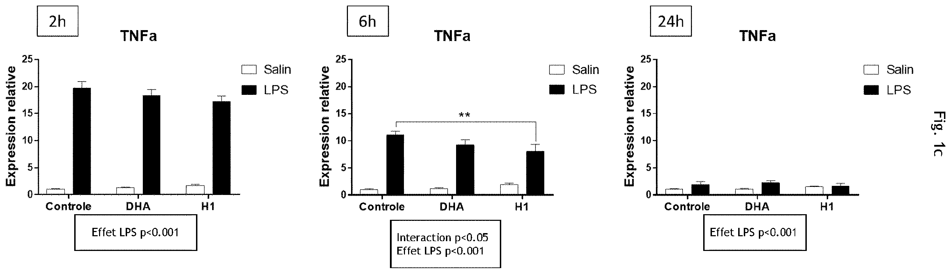

Les résultats montrent que dans la condition contrôle, le LPS induit un stress inflammatoire avec une forte expression d'IL-6, d'IL-1β et de TNF-α. Le DHA, qui présente des capacités anti-inflammatoires, permet de réduire l'expression d'IL-6 et d'IL-1β induite par le LPS. De manière surprenante, le traitement par l'hydrolysat H1 diminue l'expression d'IL-6 et d'IL-1β induite par le LPS, avec un effet plus important que le DHA (Figures 1a et 1b). L'hydrolysat H1 diminue également l'expression de TNF-α (Figure 1c).

### 2.2. Effet de l'hydrolysat H2 sur la neuroinflammation des cellules microgliales BV2

Afin de mimer un système d'interaction cellulaire plus complexe, l'hydrolysat H2 a été testé sur un système de co-culture entre les cellules microgliales et les cellules neuronales (BV2-HT22). Un stress inflammatoire a été induit dans des cellules microgliales BV2 par le liposaccharide (LPS) selon le protocole décrit à l'Exemple 1.1. L'hydrolysat protéique H2 de l'invention a alors été testé pour sa capacité à diminuer l'expression des cytokines pro-inflammatoires IL-6, IL-1β et TNF-α dans ces cellules, selon le protocole décrit à l'Exemple 1.4.5.

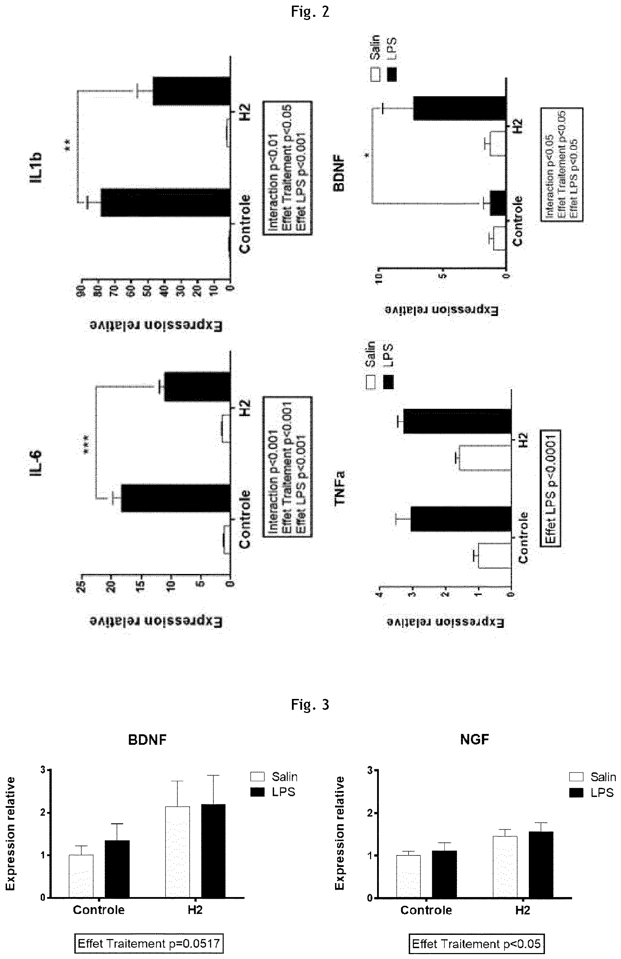

Les résultats montrent que le LPS induit l'expression des cytokines pro-inflammatoires IL-6, IL-1β et TNF-α. L'hydrolysat H2 permet de diminuer de manière significative l'expression d'IL-6 et d'IL-1β induite par le LPS 6h post-traitement (Figure 2).

### 2.3. Effet de l'hydrolysat H2 sur la neuroprotection des cellules microgliales BV2

L'hydrolysat protéique H2 de l'invention a été testé pour sa capacité à promouvoir la neuroprotection dans les cellules microgliales dans un système de co-culture BV2-HT22 (décrit à l'Exemple 1.1), notamment *via* une augmentation de l'expression du gène BDNF (Brain-Derived Neurotrophic Factor), un facteur de croissance impliqué dans la communication neuronale et l'homéostasie, selon le protocole décrit à l'Exemple 1.4.5.

Les résultats ne montrent pas d'effet du LPS sur l'expression de BDNF à 6h. Par contre, on constate que le traitement par l'hydrolysat H2 induit son expression dans des conditions inflammatoires (Figure 2). Une supplémentation en hydrolysat H2 facilite la mise en place d'une neuro-protection en condition inflammatoire.

Dans leur ensemble, les différentes données obtenues sur l'expression des cytokines pro-inflammatoires et des marqueurs neurotrophiques montrent que l'hydrolysat de l'invention possède une activité anti-inflammatoire et neuro-protectrice.

### 2.4. Effet de l'hydrolysat H2 sur la neuroprotection des cellules neuronales HT22

L'hydrolysat protéique H2 de l'invention a été testé pour sa capacité à promouvoir la neuroprotection dans les cellules neuronales (cellules HT22) dans un système de co-culture BV2-HT22, (décrit à l'Exemple 1.1). En plus de l'expression de BDNF, l'expression du gène NGF (Nerve Growth Factor), un facteur de croissance neuronal, a également été étudiée selon le protocole décrit à l'Exemple 1.4.5.

Les résultats montrent que le LPS n'a pas d'effet sur l'expression de BNDF et NGF mais que le traitement par l'hydrolysat H2 augmente leur expression à la fois en condition témoin (saline) et inflammatoire (LPS), ce qui suggère que l'hydrolysat de l'invention possède une activité neuroprotectrice (Figure 3).

### Exemple 3 : Effet in vivo de l'hydrolysat

Pour ces tests *in vivo,* les animaux ont été élevés, nourris et répartis en différents groupes selon le protocole décrit à l'Exemple 1.2.

### 3.1. Effet de l'hydrolysat H2 sur le comportement de type anxieux

La réponse au stress met en jeu l'axe hypothalamo-hypophyso-surrénalien (axe HPA). En présence d'un stress, le cerveau entraine, par l'intermédiaire d'hormones, la sécrétion de cortisol chez l'Homme et corticostérone chez la souris, des hormones de stress ayant une influence sur le système immunitaire et permettant le retour à l'homéostasie. Une fois ce retour à l'homéostasie réalisé, le cortisol agit par rétrocontrôle négatif sur le cerveau permettant de diminuer sa propre sécrétion.

Chez les personnes souffrant d'anxiété, ce qui représente 10% des personnes âgées, cet axe est perturbé entrainant des dommages au niveau du cerveau, notamment au niveau de l'hippocampe.

Les effets d'une supplémentation en hydrolysat protéique selon l'invention sur le comportement de type anxieux ont été étudiés au moyen du test dit de l'open-field (OF). Le fonctionnement de ce test est décrit à l'Exemple 1.3.2.

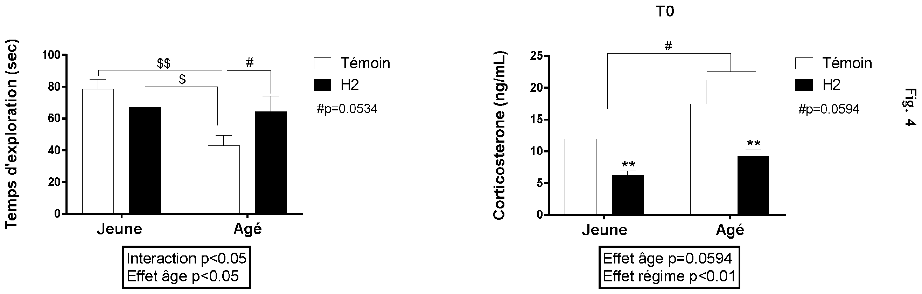

Les résultats de la Figure 4 représentent le temps passé au centre de l'OF. Comme attendu, les animaux âgés passent moins de temps au centre du dispositif que les animaux jeunes, ce qui traduit un comportement de type anxieux. Cependant, les animaux âgés ayant reçu de l'hydrolysat H2 n'ont pas un temps d'exploration au centre du test significativement différent de celui des animaux jeunes supplémentés ou non. La supplémentation en H2 permet donc de prévenir les comportements de type anxieux chez les animaux âgés et de maintenir un niveau similaire à celui des animaux jeunes.

Les résultats de ce test ont été associés au dosage de corticostérone à l'état basal (selon le protocole décrit à l'Exemple 1.4.1). On constate (Figure 4) que dans la population témoin, les animaux âgés présentent des taux de corticostérone plus importants que les animaux jeunes. Dans la population supplémentée en hydrolysat, on constate que les taux de corticostérone sont équivalents chez les animaux jeunes et chez les animaux âgés.

Ainsi, chez les animaux âgés, la supplémentation en hydrolysat permet un retour des taux de corticostérone à l'état basal.

### 3.2. Effet de l'hydrolysat H2 sur la réactivité au stress

Il est connu que les personnes âgées présentent une altération des capacités d'adaptation et des difficultés à gérer les petits stress du quotidien. Les effets d'une supplémentation en hydrolysat sur la réactivité au stress ont ainsi été testés.

Pour cela un stress modéré a été induit chez des souris par une contention de 30 minutes. Le fonctionnement de ce test est décrit à l'Exemple 1.3.6. Puis, un prélèvement de sang a été effectué à 30, 60 et 90 minutes afin de déterminer la cinétique d'expression de la corticostérone en réponse au stress (selon le protocole décrit à l'Exemple 1.4.1).

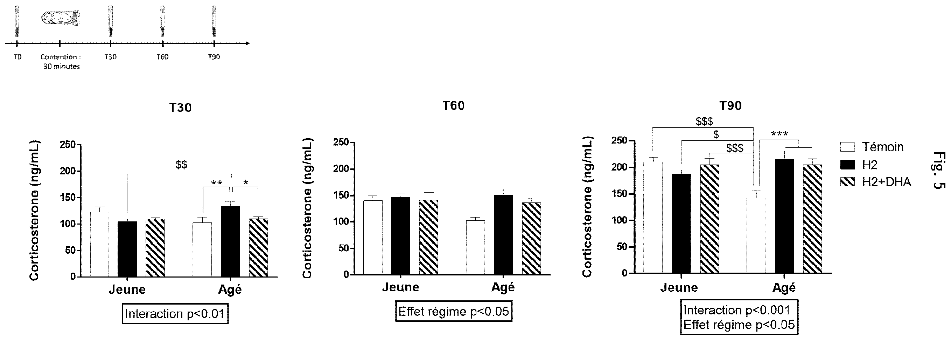

Les résultats de la Figure 5 montrent qu'à 30 min, il n'y a pas d'effet âge ni d'effet supplémentation, mais une interaction [âge x supplémentation] significative a été mise en évidence. En effet, à 30 min, les souris témoins âgées secrètent moins de corticostérone après le stress que les souris âgées supplémentées avec l'hydrolysat, suggérant une altération de la réponse au stress. A 90 min, une interaction [âge x supplémentation] significative a été mise en évidence. En effet, à 90 min, les souris âgées témoins secrètent moins de corticostérone après le stress de contention par rapport aux souris jeunes témoins, suggérant une altération de la réponse au stress. De plus, la supplémentation en hydrolysat permet de restaurer des niveaux de corticostérone chez les souris âgées similaires à ceux des souris jeunes et de prévenir cette altération de la réponse au stress liée à l'âge. Par ailleurs, les mêmes résultats sont observés lorsque les animaux sont supplémentés par une combinaison d'hydrolysat et de DHA (Figure 5).

Ainsi, la supplémentation en hydrolysat, complémenté ou non en DHA, chez les animaux âgés permet de restaurer une réactivité au stress similaire à celle des souris jeunes.

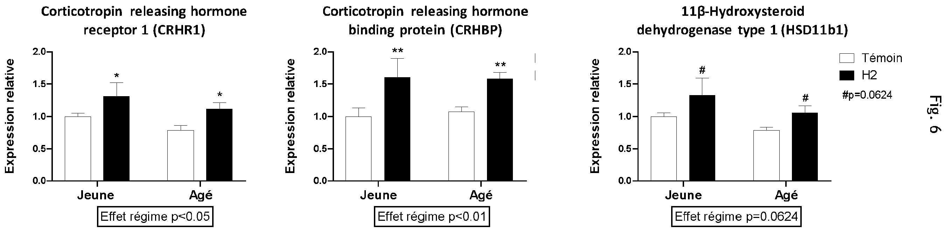

Pour aller plus loin, l'expression des gènes impliqués dans la réponse au stress au niveau de l'hypothalamus a été quantifiée (selon le protocole décrit à l'Exemple 1.4.5). La Figure 6 montre que l'âge n'a pas d'impact sur l'expression des gènes CrhR1, CRHBP et HSD11β1. Par contre, la supplémentation en hydrolysat augmente de façon significative l'expression de CrhR1 et CRHBP et tend à augmenter l'expression de HSD11β1, suggérant que les souris supplémentées en hydrolysat ont une modulation de l'expression des gènes de réponse aux stress plus importante.

### 3.3. Effet de l'hydrolysat H2 sur la mémoire hippocampique

La mémoire de travail et la mémoire épisodique, souvenirs autobiographiques impliquant une notion spatiale, sont les formes de mémoire les plus touchées au cours du vieillissement. Chez l'animal, la mémoire épisodique n'est pas évaluable en tant que telle. Elle est donc modélisée par l'étude de la mémoire spatiale.

### 3.3.1. Effet de l'hydrolysat H2 sur la mémoire hippocampique à court terme

Le test du labyrinthe en Y (décrit à l'Exemple 1.3.1) permet d'évaluer la mémoire de travail spatiale à court terme et implique la voie hippocampo-préfrontale.

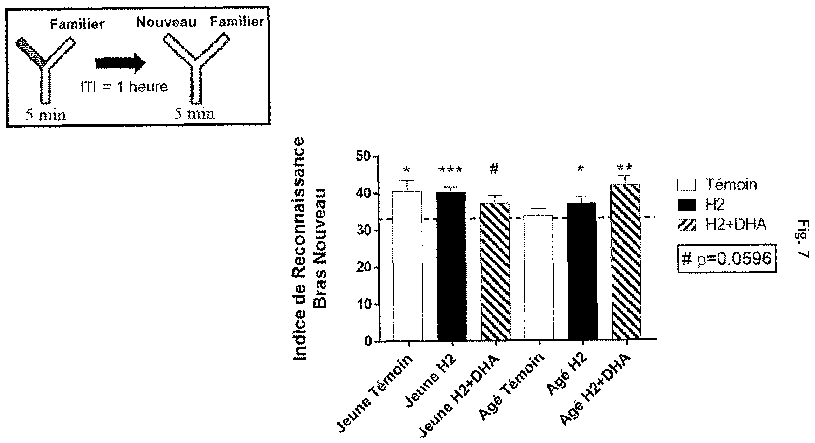

Les résultats de la Figure 7 représentent l'indice de reconnaissance du bras nouveau.

Les résultats montrent que les souris jeunes reconnaissent le bras nouveau quelle que soit la supplémentation, puisqu'elles explorent préférentiellement ce bras de façon significativement différente à celle obtenue par le hasard (33%). Les animaux âgés témoins présentent une altération de leur capacité mnésique puisqu'ils n'explorent pas le bras nouveau de façon différente à une exploration due au hasard. La supplémentation en hydrolysat H2 permet de maintenir les capacités mnésiques des animaux âgés de façon significative (Figure 7).

Les mêmes résultats sont observés lorsque les animaux sont supplémentés par l'hydrolysat complémenté en DHA (Figure 7).

Ainsi, la supplémentation en hydrolysat, complémenté ou non en DHA, chez les animaux âgés permet de prévenir les déficits de mémoire dépendante de l'hippocampe.

### 3.3.2. Effet de l'hydrolysat H2 sur la mémoire hippocampique à long terme

Le test du labyrinthe aquatique de Morris (décrit à l'Exemple 1.3.3) permet d'évaluer à la fois l'apprentissage et la mémoire.

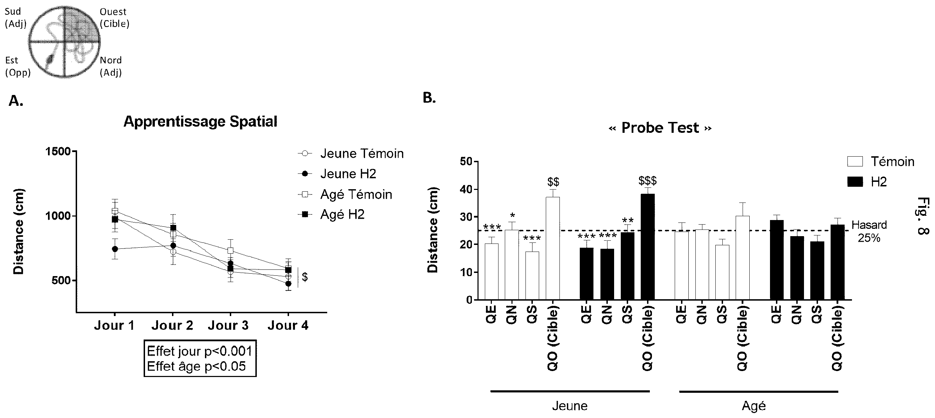

Concernant l'apprentissage spatial, toutes les souris, jeunes ou âgées, supplémentées ou non, ont appris la localisation de la plateforme puisque la distance parcourue pour atteindre la plateforme diminue au cours des jours d'apprentissage. Cependant, les souris âgées parcourent plus de distance pour atteindre la plateforme, révélant un déficit d'apprentissage spatial (Figure 8).

Le probe test (décrit à l'Exemple 1.3.4) permet de tester la mémoire spatiale de référence des animaux. Il a été réalisé 72h après le dernier jour d'apprentissage.

Lors de ce test, on distingue 4 quadrants, le quadrant opposé (est) correspond au point d'introduction des souris dans la piscine, les quadrants adjacents (nord et sud), et le quadrant cible (ouest) qui correspond à l'endroit où se trouvait précédemment la plateforme.

Les résultats montrent que les souris jeunes parcourent plus de distance dans le quadrant cible que dans les autres quadrants. Ces souris se rappellent donc de la localisation de la plateforme. A l'inverse, les souris âgées, présentant un trouble de l'apprentissage spatial, parcourent autant de distance dans les 4 quadrants. On peut donc en conclure que ces souris ne se rappellent plus de la localisation de la plateforme. Chez les souris âgées supplémentées en hydrolysat, on constate que l'altération des capacités mnésiques n'est pas récupérée (Figure 8).

Ainsi, nous n'observons pas de différence d'apprentissage entre les animaux jeunes et âgés mais les animaux âgés présentent des altérations cognitives quel que soit le régime. Cependant, une analyse plus fine des stratégies d'apprentissage (décrit à l'exemple 1.3.5) montre des effets positifs de l'hydrolysat.

En effet, pour atteindre la plateforme située dans la piscine, les souris se repèrent grâce à des indices spatiaux fixés sur les murs de la pièce. Au cours de l'apprentissage, les souris passent de stratégies non spatiales à des stratégies spatiales, plus élaborées. Des différences entre les groupes quant à l'utilisation des stratégies spatiales jour par jour ont donc été recherchées.

Il a été constaté que les souris témoins ont progressé vers une stratégie spatiale au fur et à mesure des jours d'apprentissage, avant d'atteindre un palier chez les jeunes. Pour les groupes supplémentés, on constate l'utilisation plus importante de stratégies spatiales dès le 1er jour, ce qui montre un effet bénéfique de l'hydrolysat (résultats non montrés).

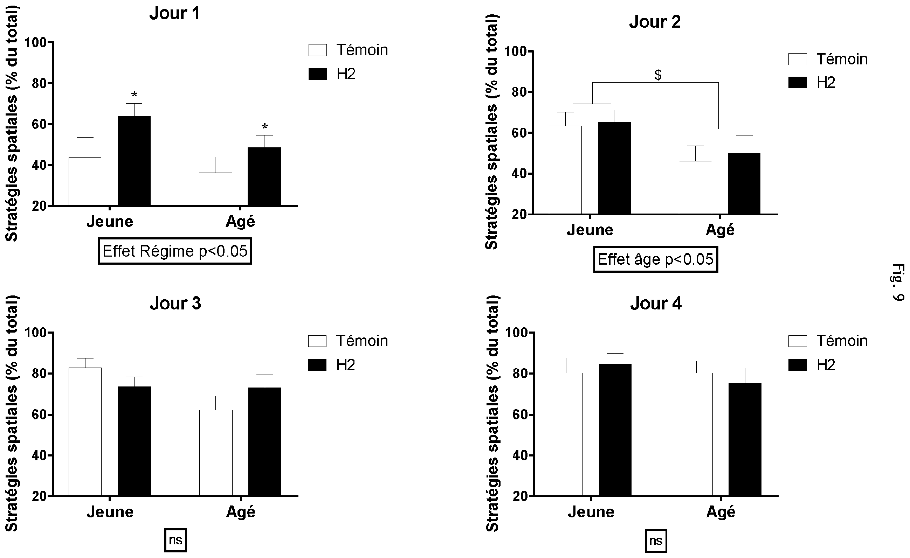

L'utilisation des stratégies spatiales entre les groupes a également été comparée jour par jour (Figure 9).

Au jour 1 : les souris supplémentées en hydrolysat utilisent plus de stratégies spatiales.

Au jour 2 : Les effets liés à la supplémentation en hydrolysat ne sont plus observés, cependant les souris âgées utilisent moins de stratégies spatiales que les souris jeunes.

Aux jours 3 et 4 : Les différences entre les groupes ne sont plus observées ce qui montre que les souris âgées utilisent autant de stratégies spatiales que les souris jeunes.

Ainsi, la supplémentation en hydrolysat augmente l'utilisation des stratégies spatiales au 1er jour.

En conclusion, l'ensemble de ces tests *in vivo* montre que la supplémentation en hydrolysat a des effets bénéfiques sur la réactivité au stress d'une part et sur la mémoire à court terme dépendante de l'hippocampe d'autre part.

### Exemple 4 : Analyses biochimiques des effets de l'hydrolysat H2 sur la neuroinflammation hippocampique

En condition physiologique, les microglies, qui sont les cellules immunocompétentes du cerveau, assurent le maintien de l'homéostasie en surveillant l'environnement. Ces cellules expriment CD11b. En présence d'un stress ou d'une agression, l'agent pathogène (ici le LPS) se fixe sur son récepteur (TLR4) exprimé par les cellules microgliales, ce qui entraine l'activation des microglies qui expriment alors Iba1. L'activation des microglies entraine l'activation de la voie NFkB et donc la production de cytokines pro-inflammatoires telles que l'IL-6, l'IL-1β et le TNF-α. La libération de ces cytokines pro-inflammatoires perturbe la communication neuronale, ce qui peut endommager les neurones environnants. Les neurones sécrètent alors des facteurs de croissances neuronaux (NGF, BDNF) qui inhibent NFkB et donc l'inflammation, ce qui contribue au retour à l'homéostasie.

Lors du vieillissement, une inflammation chronique à bas bruit s'installe. Elle se caractérise par la hausse du nombre de microglies et de la réactivité microgliale et par conséquent la libération plus importante de cytokines pro-inflammatoires à l'état basal.

Ainsi, l'expression des marqueurs microgliaux Cd11b et Iba1, a été étudiée lors du vieillissement (selon le protocole décrit à l'Exemple 1.4.5).

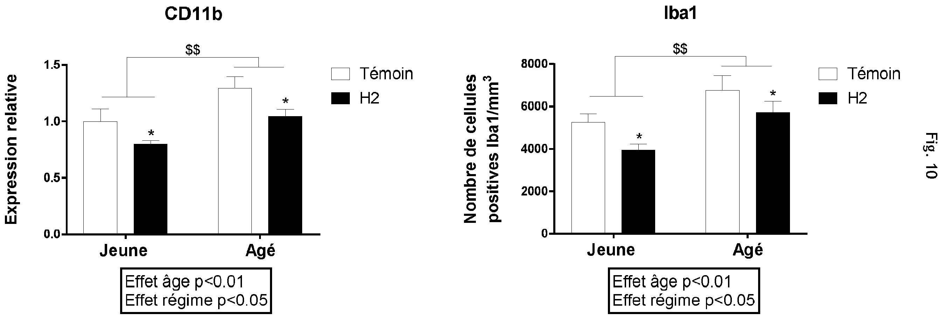

Les résultats montrent que les souris âgées expriment de façon plus importante Cd11b et Iba1, marqueurs d'une inflammation chronique à bas bruit. De façon intéressante, la supplémentation en hydrolysat diminue de façon significative leur expression, donc diminue l'activation microgliale, ce qui est bénéfique pour les animaux âgés (Figure 10).

En conclusion, la supplémentation en hydrolysat a des effets bénéfiques sur la neuroinflammation hippocampique.

### Exemple 5 : Effet in vivo de l'hydrolysat H2 sur le métabolisme énergétique hippocampique au cours du vieillissement (inflammation chronique)

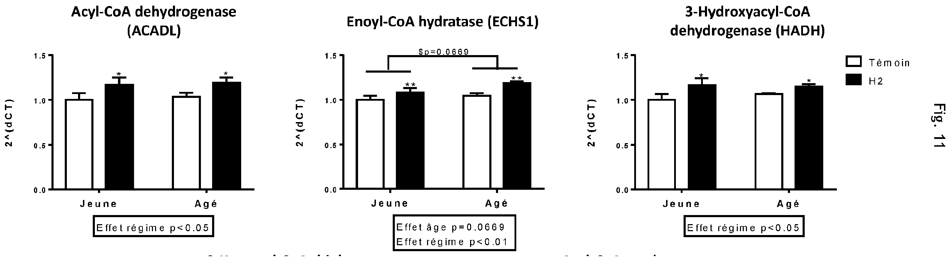

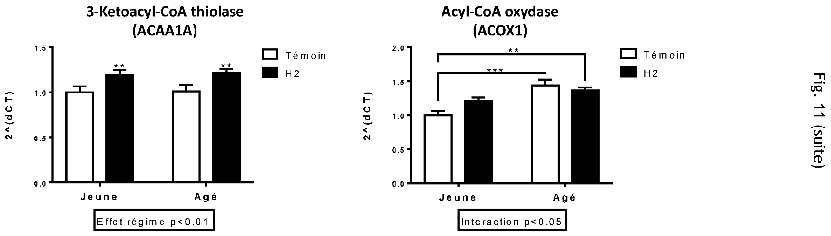

Les mitochondries et les péroxysomes sont des organites jouant un rôle important dans le métabolisme énergétique cellulaire. Ils sont distribués au niveau des différents types cellulaires (neurones, microglies...). Les mitochondries sont considérées comme la « centrale énergétique » et génèrent de l'ATP impliqué dans la maintenance et la réparation cellulaire et nécessaire à certaines fonctions comme la neurotransmission dans le cas des neurones. Par un mécanisme similaire à la mitochondrie, les peroxysomes (organites cellulaires participant principalement à la détoxification cellulaire) réalisent également la β-oxydation des acides gras à longue chaine. Les résultats (selon le protocole décrit à l'Exemple 1.4.6) montrent que les enzymes impliquées dans la β-oxydation mitochondriale et peroxysomale sont impactées par la supplémentation en hydrolysat (Figure 11). En effet, l'expression de l'acyl-CoA déshydrogénase, l'enoyl-CoA hydratase, la 3-hydroxyacyl-CoA déshydrogénase et la 3-ketoacyl-CoA thiolase (impliquées respectivement dans la 1^{ère}, 2^{ème}, 3^{ème} et dernière étape de la β-oxydation mitochondriale) est augmentée par la supplémentation. Une interaction entre l'âge et la supplémentation a été mise en évidence pour l'expression de l'acyl-CoA oxydase (impliquée dans la 1ère étape de la β-oxydation peroxysomale). De manière inattendue, l'hydrolysat H2 a permis d'amplifier l'expression des gènes des enzymes impliquées dans le métabolisme cellulaire.

### Exemple 6 : Effet in vivo de l'hydrolysat H2 sur les défenses anti-oxydantes hippocampiques au cours du vieillissement (inflammation chronique)

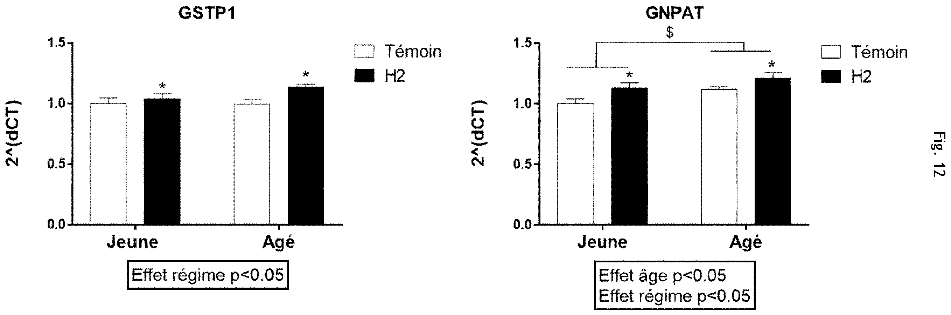

Au cours du vieillissement, les cellules tendent à accumuler des molécules agrégées dysfonctionnelles résultant d'un déséquilibre oxydatif : il est observé une augmentation de la production d'espèces réactives de l'oxygène et/ou une diminution des défenses anti-oxydantes. Nous avons évalué l'effet de l'hydrolysat sur la glutathion S-transferase qui est une enzyme de défense anti-oxydante et sur la glycéronephosphate O-acyltransferase qui est impliquée dans la 1^{ère} étape de la synthèse des plasmalogènes, lipides ayant un rôle antioxydant. Les résultats (obtenus selon le protocole décrit à l'Exemple 1.4.6) montrent que la supplémentation en hydrolysat augmente l'expression des gènes de ces enzymes, suggérant un effet positif de l'hydrolysat sur les défenses anti-oxydantes (Figure 12).

### Exemple 7 : Effet in vivo de l'hydrolysat H2 sur la neuroinflammation hippocampique en réponse à une inflammation aigue

L'effet de l'hydrolysat a été évalué sur l'inflammation aigue (induite par le LPS) sur des souris supplémentées pendant 18 jours avec de l'hydrolysat ou du DHA (selon le protocole décrit à l'Exemple 1.4.5).

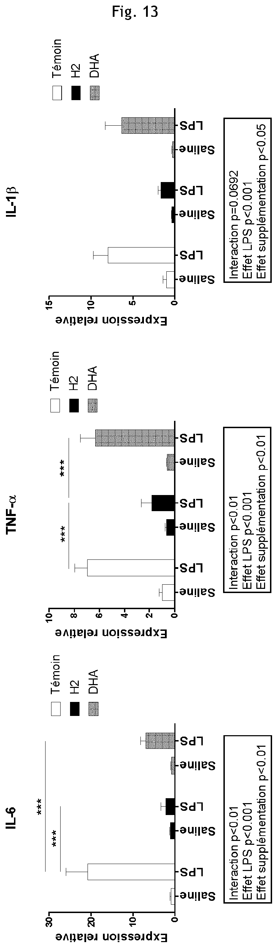

Les modifications de l'expression des cytokines pro-inflammatoires en réponse au LPS (Figure 13) ont ensuite été évaluées. Comme attendu, le LPS a significativement augmenté l'expression d'IL-6, de TNF-α, d'IL-1β. Les supplémentations ont significativement modulé l'expression des cytokines pro-inflammatoires (IL-6, TNF-α et IL-1β). Une interaction LPS x supplémentation a été mise en évidence pour IL-6 et TNF-α ainsi qu'une tendance pour IL-1B. En effet, chez les animaux traités au LPS, l'expression d'IL-6 a été significativement diminuée par les supplémentations en hydrolysat et en DHA, tandis que celle de TNF-α a été diminuée par la supplémentation en hydrolysat.

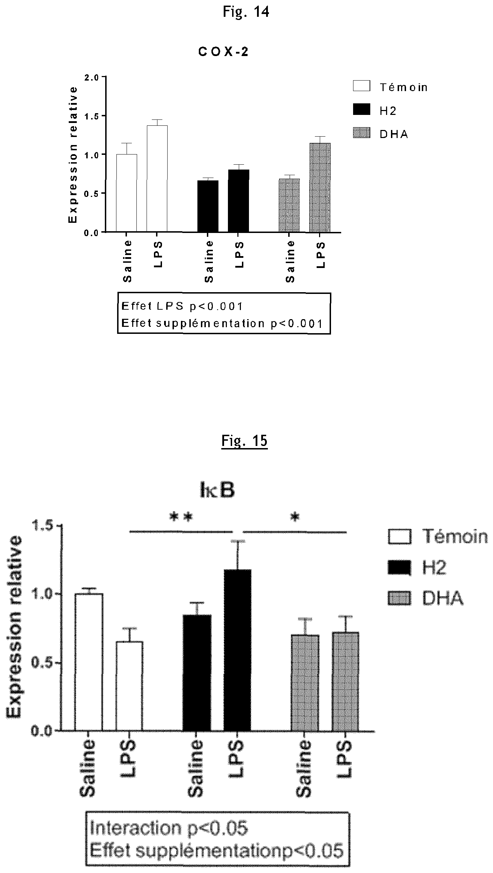

L'expression de COX-2 (selon le protocole décrit à l'Exemple 1.4.5), impliqué dans la synthèse de médiateurs lipidiques de l'inflammation a également été déterminée. Son expression a été significativement augmentée par le traitement au LPS et significativement diminuée par les supplémentations (Figure 14).

L'expression protéique d'IkB (selon le protocole décrit à l'Exemple 1.4.7) impliqué dans la régulation de l'expression de ces facteurs inflammatoires a été évaluée (Figure 15). Les supplémentations ont un effet significatif sur l'expression d'IkB (qui est un inhibiteur de NFkB, lui-même responsable de la synthèse des facteurs inflammatoires) et une interaction entre les supplémentations et le LPS a été mise en évidence. En effet, en condition inflammatoire, l'expression d'IkB est significativement augmentée par la supplémentation en hydrolysat.

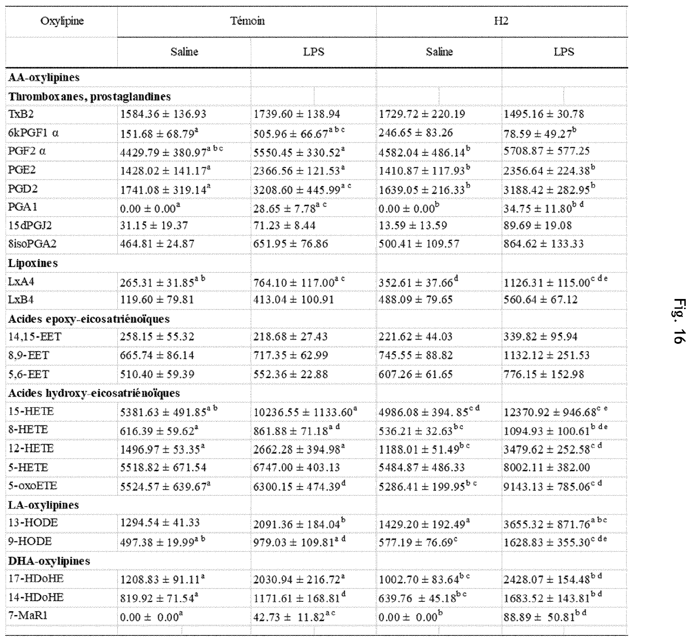

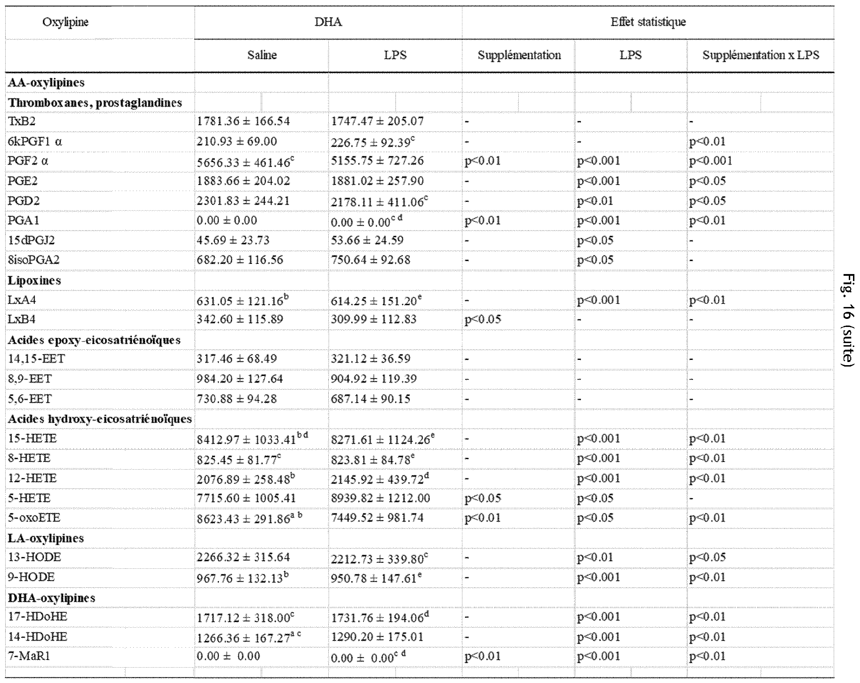

Les AGPI peuvent être convertis en dérivés lipidiques bioactifs, ou oxylipines, contribuant à leurs propriétés immunomodulatrices. Les dérivés des AGPI n-6 sont pour la plupart pro-inflammatoires tandis que ceux dérivés des AGPI n-3 sont anti-inflammatoires. L'impact de la supplémentation sur la composition en acides gras (selon le protocole décrit à l'Exemple 1.4.8) et sur la concentration en oxylipines dérivées des AGPI n-6 et n-3 dans l'hippocampe (selon le protocole décrit à l'Exemple 1.4.9) après traitement au NaCl ou au LPS a été évalué. Il a pu être montré en 1^{er} lieu que les supplémentations ont un impact sur la composition en AGPI au niveau du cortex : les supplémentations augmentent les taux d'AGPI n-3 et diminuent les taux d'AGPI n-6. Presque tous les AGPI sont impactés : 20 :3 n-6, 20 :4 n-6, 22 :4 n-6, 22 :5 n-6, 20 :5 n-3 et 22 :6 n-3. Les supplémentations modulent les concentrations de dérivés de l'acide arachidonique et du DHA (Figure 16). De plus, une interaction entre les facteurs LPS et supplémentation a été mise en évidence pour des oxylipines dérivées de l'acide arachidonique et dérivées du DHA. En effet, concernant les dérivés des AGPI n-6, en condition saline, la supplémentation en hydrolysat a augmenté la concentration de PGF2 α comparé au groupe témoin. Chez les animaux traités au LPS, la supplémentation en hydrolysat a augmenté la concentration de PGE2 (p<0,01), PGD2 (p<0,01), PGA1 (p<0,001), 15-HETE (p<0,001), 8-HETE (p<0,001), 12-HETE (p<0,001), 5-oxoETE (p<0,001) comparé aux animaux ayant reçu la solution saline. Elle a aussi augmenté les niveaux de PGA1, LxA4, 15-HETE, 8-HETE, 5-oxoETE comparé aux témoins (LxA4 et 8-HETE : p<0,05, 5-oxoETE : p<0,01) ou aux animaux supplémentés en DHA (LxA4 et 8-HETE : p<0,05, 15-HETE : p<0,01, PGA1 : p<0,001). Concernant les dérivés du DHA, en condition inflammatoire, la supplémentation en hydrolysat a augmenté les niveaux de 14-HDoHE (p<0,001), 17-HDoHE (p<0,001) et 7-MaR1 (p<0,01) comparé aux animaux traités au NaCl et comparé aux animaux du groupe témoin (14-HDoDE : p<0,05) ou à la supplémentation en DHA (17-HDoHE : p<0,05, 7-MaR1 : p<0,001).

### Exemple 8 : Effet in vivo de l'hydrolysat H2 sur la survie neuronale en réponse à une inflammation aigue

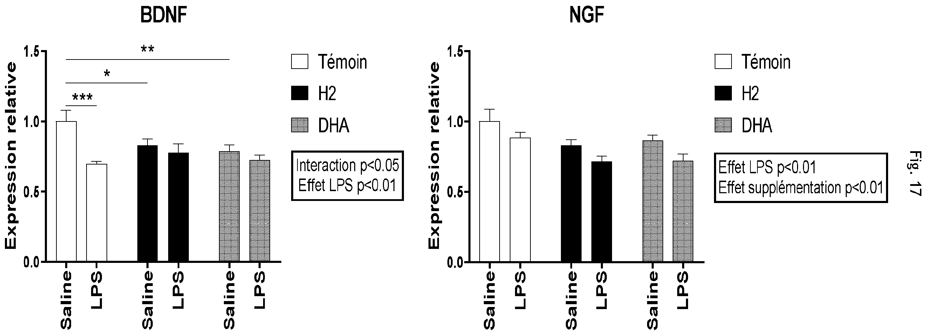

L'effet des supplémentations en hydrolysat H2 et en DHA sur l'expression des neurotrophines BDNF et NGF, selon le protocole décrit à l'Exemple 1.4.5, a été évalué (Figure 17). Les supplémentations diminuent l'expression de NGF et diminuent l'expression de BDNF en condition basale. En réponse au LPS, l'expression de BDNF est stable dans les groupes supplémentés en hydrolysat et DHA alors qu'elle diminue dans le groupe témoin.

### REFERENCES BIBLIOGRAPHIQUES

1. European Commission. 2009 Ageing Report: Economic and Budgetary Projections for the EU-27 Member States (2008-2060). Brussels: European Communities, 2009.
2. Kinsella K and He.W . An aging world, International population reports 2008. National Institute On Aging, U.S. Census Bureau, p1-191.
3. VAB Nutrition. Focus thématique : les Problématiques Santé actuelles et en émergence qui touchent ou toucheront la cible «Seniors» et l'offre nutritionnelle à développer pour en retarder ou limiter l'impact. 2009. Etude bibliographique financée par Valorial / CRITT Santé Bretagne.
4. Bousquet J, et al., Operational Définition of Active and Healthy Ageing (AHA): A Conceptual Framework. J Nutr Health Aging. 2015.19(9):955-60.
5. Banfield Pet Hospital's State of Pet Health 2013 Report. accessible sur www.banfield.com/Banfield/media/PDF/Downtoads/soph/Banfield-State-of-Pet-Health-Report_2013.pdf
6. Landsberg GM, Nichol J, Araujo JA. Cognitive dysfunction syndrome: a disease of canine and feline brain aging. Vet Clin North Am Small Anim Pract. 2012. 42(4):749-68.
7. Salvin H.E., McGreevy P.D., Sachdev P.S., Valenzuela M.J. Under diagnosis of canine dysfunction: a cross sectional survey in older companion dogs. The Veterinary Journal. 2010. 184 : 277-281.
8. De Smedt-Peyrusse V., Sargueil F., Moranis A., Harizi H., Mongrand S., Layé S. Docosahexaenoic acid prevents lipopolysaccharide-induced cytokine production in microglial cells by inhibiting lipopolysaccharide receptor présentation but not its membrane subdomain localization. J Neurochem 2008. 105:296-307.
9. Rey C., Delpech J.C., Madore C., Nadjar A., Greenhalgh AD., Amadieu C., Aubert A., Pallet V., Vaysse C., Layé S., Joffre C. Dietary n-3 long chain PUFA supplementation promotes a pro-resolving oxylipin profile in the brain. Brain Behav Immun 2019. 76:17-27.
10. Mingam R, Moranis A., Bluthé RM., De Smedt-Peyrusse V., Kelley KW., Guesnet P., Lavialle M., Dantzer R., Layé S. Uncoupling of interleukin-6 from its signalling pathway by dietary n-3-polyunsaturated fatty acid deprivation alters sickness behaviour in mice. Eur J Neurosci 2008 28:1877-86. 11. Labrousse VF., Nadjar A., Joffre C., Costes L., Aubert A ., Grégoire S., Bretillon L., Layé S. Short-term long chain omega3 diet protects from neuroinflammatory processes and memory impairment in aged mice. PLoS One 2012. 7:e36861.
12. MoranisA., Delpech JC., De Smedt-Peyrusse V., Aubert A., Guesnet P., Lavialle M., Joffre C., Layé S. Long term adéquate n-3 polyunsaturated fatty acid diet protects from depressive-like behavior but not from working memory disruption and brain cytokine expression in aged mice. Brain Behav Immun 2012. 26:721-31.
13. Morris R. Developments of a water-maze procedure for studying spatial learning in the rat. Journal of Neuroscience Methods 1984. 11:47-60.
14. Bensalem J., Dudonné S., Gaudout D., Servat L., Calon F., Desjardins Y., Layé S., Lafenêtre P., Pallet V. Polyphenol-rich extract from grape and blueberry atténuâtes cognitive decline and improves neuronal function in aged mice. J Nutr Sci 2018. 7:e19.
15. Brody D.L., Holtzman D.M.. Morris water maze search strategy analysis in PDAPP mice before and after experimental traumatic brain injury. Exp Neurol 2006. 197:330-340.
16. Garthe A., Behr J., Kempermann G.. Adult-Generated Hippocampal Neurons Allow the Flexible Use of Spatially Precise Learning Strategies. PLOS ONE 2009. 4 :e5464.
17. Ruediger S., Spirig D., Donato F., Caroni P. Goal-oriented searching mediated by ventral hippocampus early in trial-and-error learning. Nature Neuroscience 2012. 15 :1563-1571..
18. Rey C., Nadjar A., Buaud B., Vaysse C., Aubert A., Pallet V., Layé S., Joffre C. Resolvin D1 and E1 promote resolution of inflammation in microglial cells in vitro. Brain Behav. Immun. 2016. 55 :249-259..
19. Folch J., Lees M., Sloane Stanley GH. A simple method for the isolation and purification of total lipids from animal tissues. J Biol Chem 1957. 226: 497-509.20. Le Faouder P., Baillif V., Spreadbury I., Motta J.P., Rousset P., Chene G., Guigne C., Terce F., Vanner S., Vergnolle N., Bertrand-Michel J., Dubourdeau M., Cenac N. LC-MS/MS method for rapid and concomitant quantification of pro-inflammatory and pro-resolving polyunsaturated fatty acid métabolites. J. Chromatogr. B Analyt. Technol. Biomed. Life Sci. 2013. 932 :123-133.
21. Adler-Nissen J. Enzymatic hydrolysis of proteins for increased solubility. J. Agric. Food Chem. 1976 24:1090-1093.
22. Nielsen, P. M.; Petersen, D.; Dambmann, C. Improved method for determining food protein degree of hydrolysis. Journal of Food Science 2001, 66, 642-646.
23. Mathieu-Resuge, et al., Trophic ecology of suspension-feeding bivalves inhabiting a north-eastern Pacific coastal lagoon: Comparison of different biomarkers. Marine environmental research, 145, 155-16 (2019).
24. F. Guérard et al., Journal of Molecular Catalysis B: Enzymatic 19-20 (2002) 489-498.

## Revendications

1. Hydrolysat protéique obtenu à partir d'au moins une source protéique issue de poissons bleus comprenant :
(I) un degré d'hydrolyse (DH) d'au moins 10%,
(II) au moins 80% de protéines solubles dans l'eau dont le poids moléculaire est inférieur à 1000 Da,
(III) au moins 0.3% de phospholipides, et
(IV) au moins 0.5% de DHA et EPA.

2. Hydrolysat protéique selon la revendication 1, **caractérisé en ce que** ladite source protéique est au moins issue de têtes de poissons bleus.

3. Hydrolysat protéique selon la revendication 1 ou la revendication 2, **caractérisé en ce que** ledit hydrolysat protéique est complémenté en acide docosahexaénoïque (DHA).

4. Hydrolysat protéique selon la revendication 1 ou la revendication 2, **caractérisé en ce qu'**il est obtenu par un procédé d'hydrolyse enzymatique comprenant les étapes de :
a. fourniture d'une source protéique ;
b. broyage de ladite source protéique ;
c. optionnellement, ajustement du pH ;
d. ajout d'au moins une enzyme d'hydrolyse ;
e. chauffage ;
f. séparation ;
g. séchage.

5. Composition alimentaire comprenant au moins un hydrolysat protéique tel que défini dans l'une quelconque des revendications 1 à 4, **caractérisée en ce qu'**il s'agit d'un aliment complet ou d'un complément alimentaire.

6. Composition alimentaire selon la revendication 5, **caractérisée en ce que** ladite composition alimentaire est un aliment fonctionnel ou nutraceutique.

7. Composition alimentaire selon la revendication 5 ou 6, **caractérisée en ce que** ladite composition alimentaire est un complément alimentaire, notamment un aliment fonctionnel ou nutraceutique pour l'Homme.

8. Composition alimentaire selon la revendication 6, **caractérisée en ce que** ladite composition alimentaire est un aliment pour animaux de compagnie, de préférence un aliment pour chiens ou chats.

9. Procédé de préparation d'un hydrolysat protéique tel que défini la revendication 1 ou la revendication 2 comprenant les étapes de :
a. fourniture d'une source protéique ;
b. broyage de ladite source protéique ;
c. optionnellement, ajustement du pH ;
d. ajout d'au moins une enzyme d'hydrolyse ;
e. chauffage ;
f. séparation ;
g. séchage.

10. Procédé de préparation d'un hydrolysat protéique selon la revendication 9, **caractérisé en ce qu'**il comprend en outre une étape de supplémentation en DHA.

11. Kit comprenant, dans un seul emballage, plusieurs contenants :
a) au moins un hydrolysat protéique tel que défini dans l'une quelconque des revendications 1 à 4 ;
b) un ou plusieurs ingrédients alimentaires pour animaux de compagnie, choisis de préférence dans le groupe consistant en protéines, peptides, acides aminés, céréales, glucides, matières grasses ou lipides, nutriments, facteurs d'appétence, digestats animaux, farine de viande, gluten, conservateurs, tensioactifs, agents texturants ou agents de texture ou agents stabilisants, agents colorants, composés de phosphate inorganique, arômes et/ou assaisonnements.
c) optionnellement au moins du DHA.

12. Composition pharmaceutique comprenant au moins un hydrolysat protéique tel que défini dans l'une quelconque des revendications 1 à 4, un véhicule pharmaceutiquement acceptable et/ou un excipient.

13. Hydrolysat protéique tel que défini dans l'une quelconque des revendications 1 à 4 ou composition pharmaceutique selon la revendication 12, pour une utilisation comme médicament.

## Patentansprüche

1. Proteinhydrolysat, das aus mindestens einer Proteinquelle aus Fettfischen gewonnen wird und Folgendes umfasst:
(I) einen Hydrolysegrad (HG) von mindestens 10 %,
(II) mindestens 80 % wasserlösliche Proteine mit einem Molekulargewicht unter 1000 Da,
(III) mindestens 0,3 % Phospholipide und
(IV) mindestens 0,5 % DHA und EPA.

2. Proteinhydrolysat nach Anspruch 1, **dadurch gekennzeichnet, dass** diese Proteinquelle mindestens aus Köpfen von Fettfischen gewonnen wird.

3. Proteinhydrolysat nach Anspruch 1 oder Anspruch 2, **dadurch gekennzeichnet, dass** diesem Proteinhydrolysat Docosahexaensäure (DHA) zugesetzt wird.

4. Proteinhydrolysat nach Anspruch 1 oder Anspruch 2, **dadurch gekennzeichnet, dass** es durch ein enzymatisches Hydrolyseverfahren gewonnen wird, das die folgenden Schritte umfasst:
a. Bereitstellung einer Proteinquelle;
b. Mahlen dieser Proteinquelle;
c. wahlweise Einstellung des pH-Werts;
d. Zusatz von mindestens einem Hydrolyseenzym;
e. Erhitzen;
f. Trennen;
g. Trocknen.

5. Nahrungsmittelzusammensetzung, die mindestens ein Proteinhydrolysat enthält, wie in einem der Ansprüche 1 bis 4 definiert, **dadurch gekennzeichnet, dass** es sich um ein Vollnahrungsmittel oder ein Nahrungsergänzungsmittel handelt.

6. Nahrungsmittelzusammensetzung nach Anspruch 5, **dadurch gekennzeichnet, dass** es sich bei dieser Nahrungsmittelzusammensetzung um ein funktionelles oder nutrazeutisches Nahrungsmittel handelt.

7. Nahrungsmittelzusammensetzung nach Anspruch 5 oder 6, **dadurch gekennzeichnet, dass** es sich bei dieser Nahrungsmittelzusammensetzung um ein Nahrungsergänzungsmittel, insbesondere ein funktionelles oder nutrazeutisches Nahrungsmittel für den Menschen handelt.

8. Nahrungsmittelzusammensetzung nach Anspruch 6, **dadurch gekennzeichnet, dass** es sich bei dieser Nahrungsmittelzusammensetzung um ein Futtermittel für Haustiere, vorzugsweise ein Futtermittel für Hunde oder Katzen handelt.

9. Verfahren zur Herstellung eines Proteinhydrolysats, wie in Anspruch 1 oder Anspruch 2 definiert, das die folgenden Schritte umfasst:
a. Bereitstellung einer Proteinquelle;
b. Mahlen dieser Proteinquelle;
c. wahlweise Einstellung des pH-Werts;
d. Zusatz von mindestens einem Hydrolyseenzym;
e. Erhitzen;
f. Trennen;
g. Trocknen.

10. Verfahren zur Herstellung eines Proteinhydrolysats nach Anspruch 9, **dadurch gekennzeichnet, dass** es außerdem einen DHA-Zusatzschritt umfasst.

11. Set, das in einer gemeinsamen Verpackung mehrere Inhaltsstoffe enthält:
a) mindestens ein Proteinhydrolysat, wie in einem der Ansprüche 1 bis 4 definiert;
b) eine oder mehrere Nahrungsmittelzutaten für Haustiere, vorzugsweise aus der Gruppe der Proteine, Peptide, Aminosäuren, Getreideprodukte, Kohlenhydrate, Fette oder Lipide, Nährstoffe, Appetitanreger, tierischen Gärreste, Fleischmehle, Glutene, Konservierungsmittel, Tenside, Strukturfestiger oder Texturierungsmittel oder Stabilisierungsmittel, Farbstoffe, anorganischen Phosphatverbindungen, Aromastoffe und/oder Würzmittel.
c) wahlweise mindestens DHA.

12. Pharmazeutische Zusammensetzung, die mindestens ein Proteinhydrolysat, wie in einem der Ansprüche 1 bis 4 definiert, einen pharmazeutisch verträglichen Träger und/oder einen Hilfsstoff umfasst.

13. Proteinhydrolysat, wie in einem der Ansprüche 1 bis 4 definiert, oder pharmazeutische Zusammensetzung nach Anspruch 12 zur Verwendung als Arzneimittel.

## Claims

1. Protein hydrolysate obtained from at least one protein source from bluefish comprising:
(I) a degree of hydrolysis (DH) of at least 10%,
(II) at least 80% of water-soluble proteins with a molecular weight of less than 1000 Da,
(III) at least 0.3% of phospholipids, and
(IV) at least 0.5% of DHA and EPA.

2. A protein hydrolysate according to claim 1, **characterised in that** said protein source is at least derived from bluefish heads.

3. Protein hydrolysate according to claim 1 or claim 2, **characterised in that** said protein hydrolysate is supplemented with docosahexaenoic acid (DHA).

4. Protein hydrolysate according to claim 1 or claim 2, **characterized in that** it is obtained by an enzymatic hydrolysis process comprising the steps of:
a. providing a protein source;
b. grinding said protein source;
c. optionally, adjusting the pH;
d. adding at least one hydrolysing enzyme;
e. heating;
f. separation;
g. drying.

5. A food composition comprising at least one protein hydrolysate as defined in any one of claims 1 to 4, **characterised in that** it is a complete food or a food supplement.

6. A food composition according to claim 5, **characterized in that** said food composition is a functional food or nutraceutical.

7. A food composition according to claim 5 or 6, **characterized in that** said food composition is a food supplement, in particular a functional food or nutraceutical for humans.

8. A food composition according to claim 6, **characterized in that** said food composition is a pet food, preferably a dog or cat food.

9. A method of preparing a protein hydrolysate as defined in claim 1 or claim 2 comprising the steps of:
a. providing a protein source;
b. grinding said protein source;
c. optionally, adjusting the pH;
d. adding at least one hydrolysis enzyme;
e. heating;
f. separation;
g. drying.

10. A process for the preparation of a protein hydrolysate according to claim 9, **characterised in that** it further comprises a DHA supplementation step.

11. A kit comprising, in a single package, a plurality of containers:
a) at least one protein hydrolysate as defined in any one of claims 1 to 4;
b) one or more pet food ingredients, preferably selected from the group consisting of proteins, peptides, amino acids, cereals, carbohydrates, fats or lipids, nutrients, palatability enhancers, animal digestates, meat meal, gluten, preservatives, surfactants, texturing, stabilising or colouring agents, inorganic phosphate compounds, flavourings and/or seasoning;
c) optionally at least DHA.

12. A pharmaceutical composition comprising at least one protein hydrolysate as defined in any one of claims 1 to 4, a pharmaceutically acceptable carrier and/or an excipient.

13. A protein hydrolysate as defined in any one of claims 1 to 4 or a pharmaceutical composition as claimed in claim 12, for use as a medicinal product.
